# EUROPEAN PATENT APPLICATION

(11) **EP 4 772 131 A1**
(43) Date of publication of application: **08.07.2026**
(21) Application number: 25227593.8
(22) Date of filing: 30.12.2025
(51) Int. Cl.: A61B 34/10, A61B 34/20

(54) **ACETABULAR CENTER OF ROTATION DETERMINATION AND FUNCTIONAL COMPUTED TOMOGRAPHY (CT) ALIGNMENT**

(30) Priority: 06.01.2025 US 202519010309
(71) Applicant: Globus Medical, Inc., Audubon, PA 19403 (US)
(72) Inventor: CAMERON, Hayden, PHILADELPHIA, 19129 (US); CHAPPUIS, Olivier, 1095 LUTRY (CH); DUCH, Loris, 1053 BRETIGNY-SUR-MORRENS VD (CH)
(74) Representative: Morabito, Sara

(57) **Abstract**

Functional alignment of a 3D image of pelvis includes identifying a reference points on each 2D image of weight-bearing pelvis; identifying corresponding reference points on a 3D image of pelvis in a non-weight bearing position; constructing references on the 2D images based on the reference points on each respective image, constructing a corresponding reference on the 3D image based on the reference points thereon; and aligning the corresponding references of the 3D image with the references of 2D images, to provide a functionally aligned 3D image to more accurately plan placement of an acetabular shell. Another aspect of the invention determines an acetabular center of rotation (CoR) calculations for use in navigation during a THA surgery, including obtaining a point cloud of a surface of an acetabulum with a navigated instrument during the THA surgery; creating an acquired surface of the acetabulum based on the point cloud; extracting an acetabular CoR from the acquired surface for use in alignment and registration.

## Description

### BACKGROUND OF THE INVENTION

The invention relates generally to devices, systems, and methods for use in computer-assisted, navigated surgical procedures. More particularly, the invention relates to computer-assisted devices, systems, and methods for planning and performing computer-assisted, navigated total hip arthroplasty (THA) surgical procedures.

Hip arthroplasty, or hip replacement, is a surgical procedure used to resurface and reconstruct a hip joint that has been damaged by disease or injury, e.g., by arthritis or a fracture. THA devices replace both the acetabulum and the femoral head that collectively comprise the hip joint. An acetabular implant is secured to the acetabulum, forming a replacement articulating surface which interfaces with the femoral implant secured to the end of the femur. The femoral implant is pivotably coupled to the acetabular implant, thereby reconstructing the hip joint. Exemplary acetabular implants are disclosed in, e.g., US Patent Application No. 17/024,876, filed September 18, 2020 (published as US 2022/0087823 A1), which is incorporated by reference as though fully set forth herein.

Robotic surgery systems including computer-assisted navigation have become a well-established technique in operating rooms, including their use in arthroplasty procedures. Computer-assisted navigation systems provide surgeons with computerized visualization of how a surgical instrument or other device that is posed relative to a patient correlates to a pose relative to medical images of the patient's anatomy, and how those poses correlate to a pre-operative surgical plan. Camera tracking systems for computer assisted surgery navigation typically use a set of tracking cameras to track a pose of a reference element on the surgical instrument, which may be coupled to a surgical robot and may be positioned by a surgeon during surgery, relative to a patient reference element (or "dynamic reference base" (DRB)) affixed to the patient. A computer model of a real instrument is associated with a reference element, so that the computer model can be overlaid on registered images of patient's anatomy. The camera tracking system uses the relative poses of the reference elements to determine how the real instrument is posed relative to the patient and to determine how the computer model of the real instrument is to be correspondingly posed as overlaid on the medical images. The surgeon can thereby use real-time visual feedback of the relative poses to navigate the surgical instrument during a surgical procedure on the patient.

As noted above, a robotic system may be used for arthroplasty procedures. The robotic system (or, "robot" or "surgical robot") has a serial arm on which an end effector is mounted. The surgeon (or "user") may hold the end effector or any instruments coupled thereto, to perform surgical operations while watching in real time on a navigation system (e.g., on stand-alone display(s) or an Augmented Reality (AR) headset), and to receive various types of relevant feedback and information associated with a defined plan for and/or progress of the surgical procedure.

The serial arm can move through computer guided control to a suitable position for the surgery, e.g., pursuant to the surgeon's request, which may be provided via a foot pedal, touchscreen, AR interaction, etc. The passive robotic structure allows the surgeon to precisely perform each operation in the procedure.

Various workflows can be available for use with the system. Such workflows may incorporate preoperative scans or images of the patient (e.g., x-ray or Computerized Tomography (CT)). On the other hand, other workflows may be imageless, and may not require any pre-operative images. Some workflows may incorporate acquisition of intra-operative information about the patient anatomy. In one example, the surgeon may measure key parameters of the bone using a camera tracking system and an appropriate tracked instrument to capture points on patient anatomy. Later, this information, and other intra-operatively acquired information may be used to plan the implant position and orientation with respect to patient anatomy, and to navigate the robot and surgical instruments during the surgical procedure.

In some workflows, the surgeon may rigidly attach a reference element to one or more bones, where the reference element includes fiducials which are detected by tracking cameras for computer assisted navigation. The reference elements allow tracking of bone position by the navigation system. The reference elements can be positioned on the bone and oriented such that they can be seen by the tracking cameras of the navigation system. Once positioned, the reference elements are attached with fixation structures (e.g., screw pins, "crocodile" jaws) on the bone (e.g., pelvis or femur). The reference elements' respective positions and orientations stay rigidly fixed with respect to the bone throughout the procedure.

Another process of various workflows is to register the patient in the tracking space of the navigation system. Patient registration can include matching the patient anatomy with a numeric representation of the corresponding bone, such as a three-dimensional (3D) model of the bone. The bone representation may be constructed from, e.g., a set of CT images (CT workflow), a set of fluoroscopy images, or based on a generic bone model (imageless workflow).

Although current surgical approaches offer sophisticated techniques in computer navigation-assisted surgeries, current approaches for registration of patient anatomical features such as, e.g., the acetabular center of rotation, and planning workflows for placement of acetabular shells may have shortcomings.

### BRIEF DESCRIPTION OF THE INVENTION

A first aspect of the disclosure provides a system for computer assisted navigation during a total hip arthroplasty (THA) surgery, comprising a computer platform including a processor and a memory, operative to obtain a point cloud of a surface of an acetabulum of a patient with a navigated instrument during the THA surgery, wherein the point cloud includes a plurality of data points detected and captured on the surface of the acetabulum by the navigated instrument; create an acquired surface of the acetabulum based on the point cloud; extract an acetabular center of rotation (CoR) from the acquired surface; and register a location of the acetabular CoR.

In certain embodiments, the computer platform is further operative to identify a position of the pelvis relative to a reference element rigidly affixed thereto.

Certain embodiments include a camera tracking system in signal communication with the computer platform, the camera tracking system being adapted to intra-operatively track a pose of the navigated instrument relative to a defined coordinate system; and a navigation controller adapted to generate navigation information for navigating the navigated instrument.

Certain embodiments include a display for displaying navigational guidance to a user via a user interface, wherein the display is in signal communication with the computer platform, wherein the navigational guidance is adapted to assist the user to obtain the point cloud.

Certain embodiments include a surgical robot having a robotic arm; and an end effector coupled to the robotic arm, wherein the end effector is adapted to receive, translate, and orient the navigated instrument; and wherein the navigation controller is adapted to control translation, rotation, and orientation of the robotic arm and the end effector.

In certain embodiments, the navigated instrument includes a stylus; an instrument reference element affixed to the stylus, wherein the instrument reference element includes a tracking array having a plurality of tracking fiducials affixed thereto; and a ball tip disposed at a distal end of the stylus, wherein each data point in the plurality of data points corresponds to a center of the ball tip during acquisition of the data point.

In certain embodiments, the point cloud is obtained by painting the surface of the acetabulum with the ball tip of the stylus, thereby acquiring the plurality of data points.

In certain embodiments, the point cloud is obtained by individually contacting a plurality of points on the surface of the acetabulum with the ball tip of the stylus, thereby acquiring each data point in the plurality of data points.

In certain embodiments, a definition of the acquired surface is independent of an orientation of the stylus relative to the surface of the acetabulum at a time of capturing of each data point in the plurality of data points.

In certain embodiments, the computer platform is further operative to collect a location of the center point of the ball tip for each data point in the acquired plurality of data points; and fit together the collected ball tip center point locations using a sphere fitting algorithm to produce a fitting sphere, wherein a surface of the fitting sphere is offset from the surface of the acetabulum by a distance corresponding to a radius of the ball tip.

In certain embodiments, the surface of the fitting sphere corresponds to the acquired surface, and the acetabular CoR corresponds to a center of the fitting sphere.

In certain embodiments, the computer platform is further operative to match the acquired surface to the surface of the acetabulum by calculating a ball tip sphere radius vector from the acquired surface to the surface of the acetabulum for the center point of the ball tip of each of the plurality of data points.

In certain embodiments, the computer platform is further operative to translate each data point along the ball tip sphere radius vector by a distance corresponding to the radius of the ball tip in a direction away from the instrument reference element on the stylus; and generate a digital model corresponding to the surface of the acetabulum by connecting the translated acquired data points.

In certain embodiments, the computer platform is further operative to identify a data point in the acquired plurality of data points as an outlier; and remove the outlier from the acquired surface of the acetabulum, wherein the acquired surface of the acetabulum is defined by a majority of the acquired plurality of data points.

In certain embodiments, the computer platform is further operative to extract an additional landmark, selected from a fitting sphere or an acetabular diameter, from the acquired surface.

A second aspect of the disclosure provides a computer program product comprising a non-transitory computer readable medium storing instructions executable by at least one processor to perform operations for computer assisted navigation during a total hip arthroplasty (THA) surgery to obtain a point cloud of a surface of an acetabulum of a pelvis of a patient with a navigated instrument during the THA surgery, wherein the point cloud includes a plurality of data points detected and captured on the surface of the acetabulum by the navigated instrument, and wherein the navigated instrument includes a stylus having an instrument reference element disposed thereon, and a ball tip disposed at a distal end of the stylus; create an acquired surface of the acetabulum based on the point cloud; extract an acetabular center of rotation (CoR) from the acquired surface; and register a location of the acetabular CoR.

Certain embodiments include instructions to collect a center point of the ball tip for each data point in the acquired plurality of data points; and fit together the collected ball tip center points using a sphere fitting algorithm to produce a fitting sphere, wherein a surface of the fitting sphere is offset from the surface of the acetabulum by a distance corresponding to a radius of the ball tip, wherein the surface of the fitting sphere corresponds to the acquired surface, and the acetabular CoR corresponds to a center of the fitting sphere.

Certain embodiments include instructions to match the acquired surface to the surface of the acetabulum by calculating a ball tip sphere radius vector from the acquired surface to the surface of the acetabulum for the center point of the ball tip of each of the plurality of data points; translate each data point along the ball tip sphere radius vector by a distance corresponding to the radius of the ball tip in a direction away from the instrument reference element on the stylus; and generate a digital model corresponding to the surface of the acetabulum by connecting the translated acquired data points.

A third aspect of the disclosure provides a method of identifying and registering an acetabular center of rotation (CoR), comprising obtaining a point cloud of a surface of an acetabulum of a pelvis of a patient with a navigated instrument during the THA surgery, wherein the point cloud includes a plurality of data points detected and captured on the surface of the acetabulum by the navigated instrument, and wherein the navigated instrument includes a stylus having an instrument reference element disposed thereon, and a ball tip disposed at a distal end of the stylus; creating an acquired surface of the acetabulum based on the point cloud; extracting an acetabular center of rotation (CoR) from the acquired surface; and registering a location of the acetabular CoR.

Certain embodiments include collecting a center point of the ball tip for each data point in the acquired plurality of data points; fitting together the collected ball tip center points using a sphere fitting algorithm to produce a fitting sphere, wherein a surface of the fitting sphere is offset from the surface of the acetabulum by a distance corresponding to a radius of the ball tip, wherein the surface of the fitting sphere corresponds to the acquired surface, and the acetabular CoR corresponds to a center of the fitting sphere; matching the acquired surface to the surface of the acetabulum by calculating a ball tip sphere radius vector from the acquired surface to the surface of the acetabulum for the center point of the ball tip of each of the plurality of data points; translating each data point along the ball tip sphere radius vector by a distance corresponding to the radius of the ball tip in a direction away from the instrument reference element on the stylus; and generating a digital model corresponding to the surface of the acetabulum by connecting the translated acquired data points.

A fourth aspect of the disclosure provides a method for planning a placement of an acetabular shell in a total hip arthroplasty (THA) procedure, comprising identifying a plurality of reference points on each of a plurality of two-dimensional (2D) images depicting a functional alignment of a pelvis of a patient in a weight-bearing position; identifying a corresponding plurality of reference points on each of a plurality of segments of a three-dimensional (3D) image depicting the pelvis in a non-weight bearing position; constructing a biomechanical reference on each of the plurality of 2D images based on the plurality of reference points on each respective image, constructing a corresponding biomechanical reference on each of the plurality of segments of the 3D image, based on the plurality of reference points on each respective segment; aligning the corresponding biomechanical references of the plurality of segments of the 3D image with the biomechanical references of the plurality of 2D images to provide a functionally aligned 3D image; and planning the placement of the acetabular shell on the functionally aligned 3D image.

In certain embodiments, the plurality of 2D images includes a 2D anterior-posterior (AP) image of the patient in a standing position; a 2D lateral image of the patient in a seated position; and a 2D lateral image of the patient in a standing position.

In certain embodiments, the plurality of reference points comprises a left and a right acetabular center of rotation (CoR), and the biomechanical reference comprises a bicoxofemoral line connecting the left and the right acetabular CoRs.

In certain embodiments, the plurality of reference points comprises a left and a right anterior superior iliac crest and a pubic symphysis, and the biomechanical reference comprises an anterior pelvic plane (APP) connecting the left and the right anterior superior iliac crests and the pubic symphysis.

Certain embodiments include, after the aligning, identifying a functional pelvic plane (FPP) of the patient on the functionally aligned 3D image; identifying a reference line on the functionally aligned 3D image, wherein the reference line is determined relative to the FPP; and planning an inclination angle of the acetabular shell relative to the reference line, wherein the reference line is a bi-ischial (BI) line.

Certain embodiments include planning a version angle of the acetabular shell relative to the FPP.

In certain embodiments, each image in the plurality of 2D images comprises an x-ray image; the 3D image comprises a computed tomography (CT) scan.

Certain embodiments include, prior to identifying the corresponding plurality of reference points, segmenting the 3D image to yield a segmented three-dimensional (3D) image, wherein each segment of the plurality of segments corresponds to a 2D slice of the CT scan.

A fifth aspect of the disclosure provides a method of aligning a three-dimensional (3D) image of a pelvis of a patient to a native functional alignment of the patient, comprising acquiring the 3D image of the pelvis in a non-weight bearing position, and segmenting the 3D image into a plurality of segments; acquiring a plurality of two-dimensional (2D) images depicting a native functional alignment of the pelvis in a plurality of weight-bearing positions; identifying a plurality of reference points on each image of the plurality of 2D images; identifying a corresponding plurality of reference points on each segment of the plurality of segments of the 3D image; constructing a biomechanical reference on each image of the plurality of 2D images based on the plurality of reference points on each respective image; constructing a corresponding biomechanical reference on each segment of the plurality of segments of the 3D image, based on the corresponding plurality of reference points on each respective segment; and aligning the corresponding biomechanical references of the plurality of segments of the 3D image with the biomechanical references of the plurality of 2D images to provide a functionally aligned 3D image.

In certain embodiments, the plurality of 2D images includes a 2D anterior-posterior (AP) image of the patient in a standing position; a 2D lateral image of the patient in a seated position; and a 2D lateral image of the patient in a standing position.

In certain embodiments, the set of reference points comprises a left and a right acetabular center of rotation (CoR), and the biomechanical reference comprises a bicoxofemoral line connecting the left and the right acetabular CoRs.

In certain embodiments, the set of reference points comprises a left and a right anterior superior iliac crest and a pubic symphysis, and the biomechanical reference comprises an anterior pelvic plane (APP) connecting the left and the right anterior superior iliac crests and the pubic symphysis.

In certain embodiments, each image in the plurality of 2D images comprises an x-ray image, the 3D image comprises a computed tomography (CT) scan, and each segment of the plurality of segments corresponds to a 2D slice of the CT scan.

A sixth aspect of the disclosure provides a system for computer assisted navigation during a total hip arthroplasty (THA) surgery, comprising a computer platform including a processor and a memory, operative to identify a plurality of reference points on each of a plurality of two-dimensional (2D) images depicting a functional alignment of a pelvis of a patient in a weight-bearing position; identify a corresponding plurality of reference points on each of a plurality of segments of a three-dimensional (3D) image depicting the pelvis in a non-weight bearing position; construct a biomechanical reference on each of the plurality of 2D images based on the plurality of reference points on each respective image; construct a corresponding biomechanical reference on each of the plurality of segments of the 3D image, based on the plurality of reference points on each respective segment; align the corresponding biomechanical references of the plurality of segments of the 3D image with the biomechanical references of the plurality of 2D images to provide a functionally aligned 3D image; and plan the placement of the acetabular shell on the functionally aligned 3D image.

These and other aspects, advantages and salient features of the invention will become apparent from the following detailed description, which, when taken in conjunction with the annexed drawings, where like parts are designated by like reference characters throughout the drawings, disclose embodiments of the invention.

### BRIEF DESCRIPTION OF THE DRAWINGS

Aspects of the present disclosure are illustrated by way of example and are not limited by the accompanying drawings. In the drawings:
FIG. 1 is an overhead view of a surgical system arranged during a surgical procedure in a surgical room which includes a camera tracking system for computer assisted navigation during surgery, and a surgical robot for robotic assistance according to some embodiments of the present disclosure.
FIG. 2 illustrates the camera tracking system and the surgical robot of FIG. 1, positioned relative to a patient according to some embodiments of the present disclosure.
FIG. 3 further illustrates the camera tracking system and the surgical robot of FIGS. 1-2, configured according to some embodiments of the present disclosure.
FIG. 4 illustrates a block diagram of a surgical system that includes an extended reality headset, a computer platform, imaging devices, and a surgical robot, which are configured to operate according to some embodiments of the present disclosure.
FIG. 5 illustrates a flowchart of a workflow during an intra-operative portion of a total hip arthroplasty (THA) surgery, in accordance with some embodiments of the present disclosure.
FIG. 6 illustrates a flowchart of a patient preparation process before registration, in accordance with some embodiments of the present disclosure.
FIG. 7 illustrates a radiographic inclination angle measured in the coronal plane of the patient, in accordance with some embodiments of the present disclosure.
FIG. 8 illustrates a radiographic version angle measured relative to the coronal plane of the patient, in accordance with some embodiments of the present disclosure.
FIG. 9 illustrates different views of landmarks and axes for registration of a functional pelvic plane (FPP) and an anterior pelvic plane (APP) of a patient, in accordance with some embodiments of the present disclosure.
FIG. 10 illustrates a reference element affixed to a pelvis of a patient, in accordance with some embodiments of the present disclosure.
FIG. 11 illustrates a method of painting the acetabulum cavity with a tracked instrument, in accordance with some embodiments of the present disclosure.
FIG. 12 illustrates a ball tip registration stylus with reflective sphere markers (left) and disk markers (right), in accordance with some embodiments of the present disclosure.
FIG. 13 illustrates one example of a user interface to guide the user, in accordance with some embodiments of the present disclosure.
FIG. 14 illustrates an exemplary schema of the surface matching algorithm, in accordance with some embodiments of the present disclosure.
FIG. 15 illustrates a palpated planar bone surface, in accordance with some embodiments of the present disclosure.
FIG. 16 illustrates a set of trial acetabular shell implants with varied diameters, in accordance with some embodiments of the present disclosure.
FIG. 17 illustrates a fully assembled inserter construct with trial acetabular shell, in accordance with some embodiments of the present disclosure.
FIG. 18 illustrates an inserter construct being inserted into the acetabulum to register the acetabular center of rotation, in accordance with some embodiments of the present disclosure.
FIG. 19 illustrates an acetabular center of rotation annotated on 2D imaging, using a reticle in accordance with some embodiments of the present disclosure.
FIG. 20 illustrates an acetabular center of rotation annotated on 3D imaging slice views, using a reticle in accordance with some embodiments of the present disclosure.
FIG. 21 illustrates a workflow for functionally aligning a 3D image, in accordance with some embodiments of the present disclosure.
FIGS. 22A and 22B illustrate standing anterior-posterior (AP) and lateral weight bearing 2D images, respectively, of a pelvis in accordance with some embodiments of the present disclosure.
FIGS. 23A, 23B, and 23C illustrate identifications of various reference points on a 3D image of a pelvis in accordance with some embodiments of the present disclosure.
FIG. 24 illustrates a lateral seated (weight bearing) 2D image of a pelvis in accordance with some embodiments of the present disclosure.
FIGS. 25A and 25B illustrate standing anterior-posterior (AP) and lateral weight bearing 2D images, respectively, of a pelvis in accordance with some embodiments of the present disclosure.
FIGS. 26 and 27 illustrate a functionally aligned 3D image of a pelvis for use in planning an acetabular shell placement in accordance with some embodiments of the present disclosure.
FIGS. 28A and 28B illustrate the contrast in acetabular shell placement planning between an as-acquired 3D image and a functionally aligned 3D image of a pelvis, in accordance with some embodiments of the present disclosure.

It is noted that the drawings of the disclosure are not necessarily to scale. The drawings are intended to depict only typical aspects of the disclosure, and therefore should not be considered as limiting the scope of the disclosure. In the drawings, like numbering represents like elements between the drawings.

### DETAILED DESCRIPTION OF THE INVENTION

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. Skilled artisans will recognize the examples provided herein have many useful alternatives and fall within the scope of the embodiments.

The present application is related to (1) US Patent Application No. 15/180,126, filed June 13, 2016 (U.S. 10,842,453), (2) US Patent Application No. 15/157,444, filed May 18, 2016 (U.S. Pub. No. 2016/0256225), (3) US Patent Application No. 18/743,685, filed June 14, 2024, (4) US Patent Application No. 18/743,388, filed June 14, 2024, (5) US Patent Application No. 18/743,647, filed June 14, 2024, (6) US Patent Application No. 18/743,615, filed June 14, 2024, (7) US Patent Application No. 18/737,123, filed June 07, 2024, (8) US Patent Application No. 18/770,993, filed July 12, 2024, (9) US Patent Application No. 18/802,689, filed August 13, 2024, (10) US Patent Application No. 18/801,924, filed August 13, 2024, (11) US Patent Application No. 18/897,137, filed September 26, 2024 (Attorney Docket No. IDR-23-152 / COXA-IDR-07), and (12) US Patent Application No. 18/909,473, filed October 8, 2024 (Attorney Docket No. IDR-24-050), each of which is incorporated herein by reference.

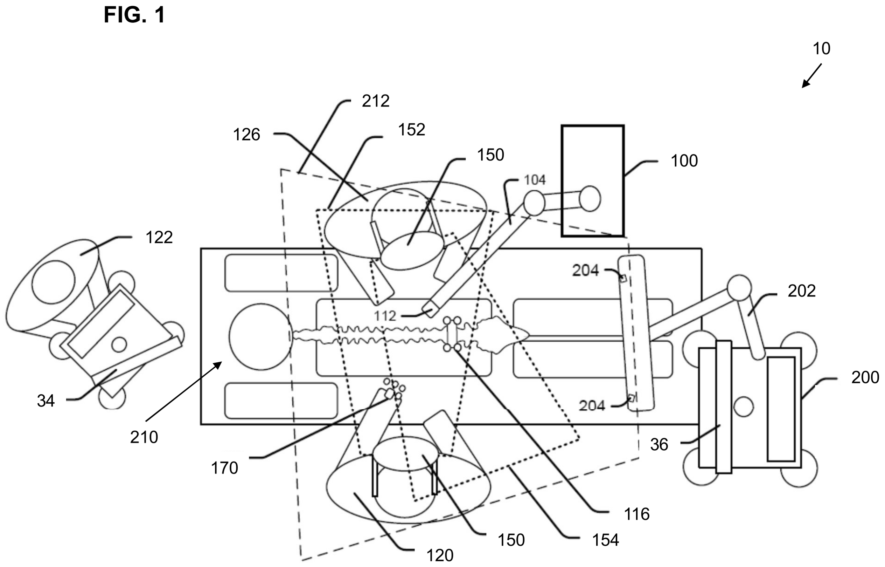

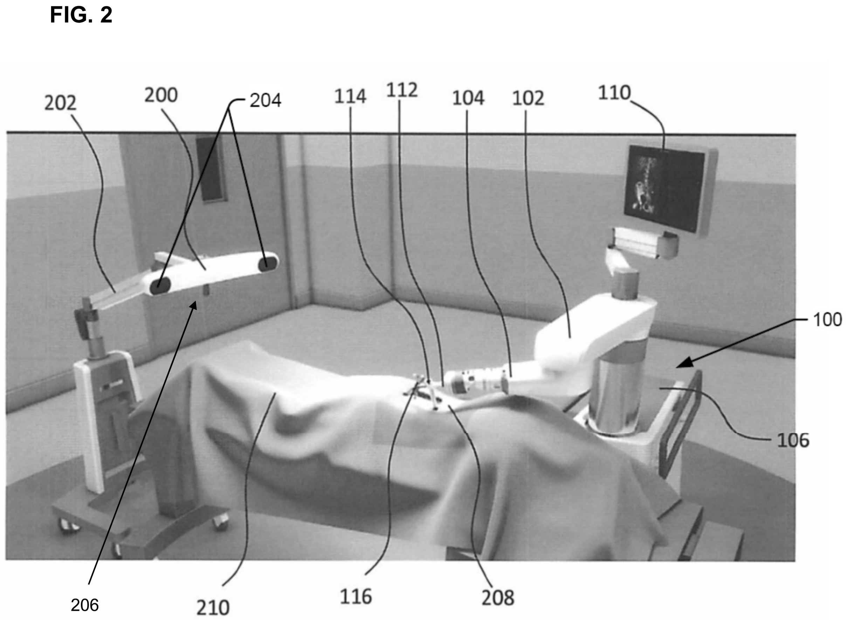

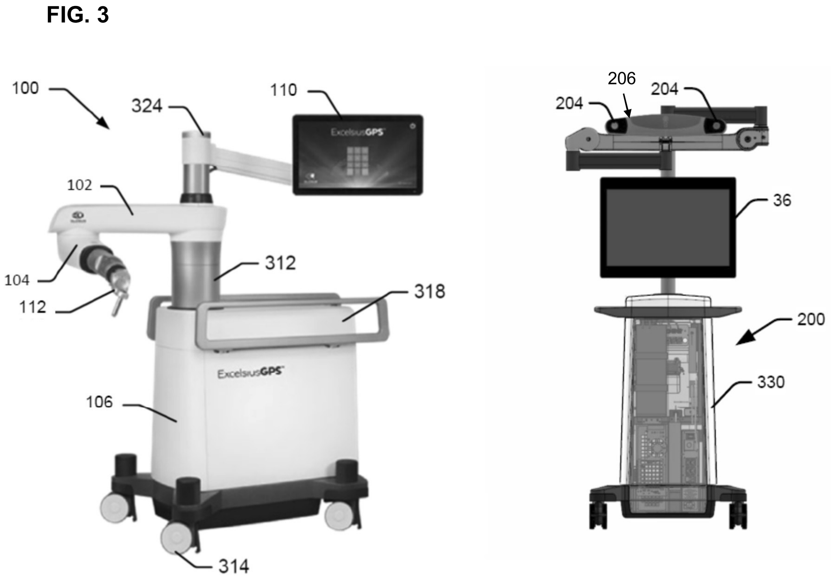

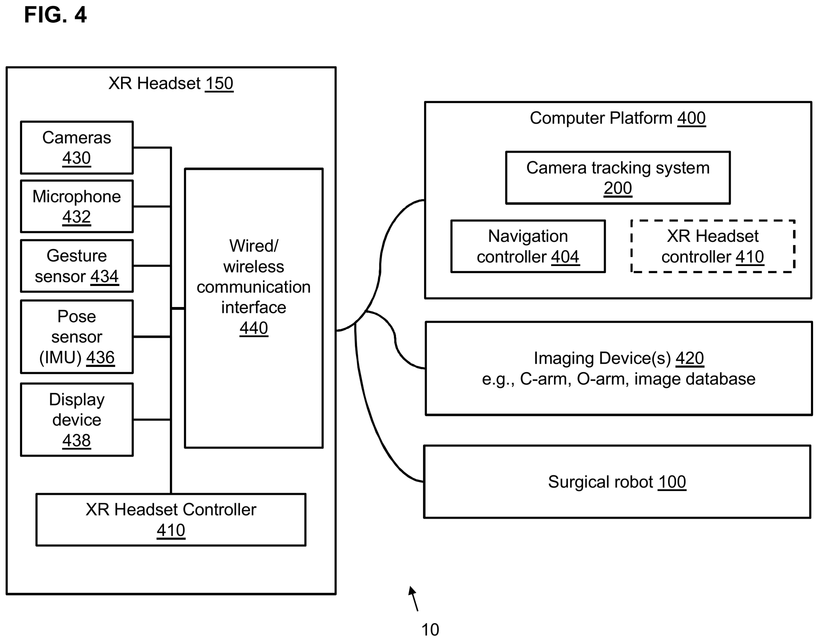

Turning to the figures, FIG. 1 is an overhead view of a surgical system 10 arranged during a surgical procedure in a surgical or operating room. The system 10 includes a camera tracking system 200 for computer assisted navigation during surgery and may further include a surgical robot 100 for robotic assistance according to some embodiments. FIG. 2 illustrates the camera tracking system 200 and the surgical robot 100 positioned relative to a patient according to some embodiments. FIG. 3 further illustrates the camera tracking system 200 and the surgical robot 100 configured according to some embodiments. FIG. 4 illustrates a block diagram of a surgical system 10 that includes an extended reality (XR) headset 150, a computer platform 400, imaging devices 420, and the surgical robot 100 which are configured to operate according to some embodiments.

The camera tracking system 200 (FIGS. 1-4) in some cases includes an intraoperative imaging system, that can include distinct imaging modalities. These imaging modalities may include one or more of fluoroscopy, 2D Radiography, and Cone-beam computed tomography (CBCT). Fluoroscopy is a medical imaging technique that shows a continuous X-ray image on a monitor, much like an X-ray movie. 2D Radiography is an imaging technique that uses X-rays to view the internal structure of a non-uniformly composed and opaque object such as the human body. CBCT (or, cone beam 3D imaging or C-arm CT), is a medical imaging technique consisting of X-ray computed tomography where the X-rays are divergent, forming a cone. The camera tracking system 200 is capable of: (1) capturing 3-Dimensional (3D) images (e.g., CT, CBCT, MCT, PET, Angiogram, MRI, ultrasound, etc.), (2) capturing 2-Dimensional (2D) images (e.g., fluoroscopy, digital radiography, ultrasound, etc.), and (3) containing an integrated or detachable navigation array having tracking markers (e.g., NIR retroreflective, NIR LED, visible, etc.), which is calibrated to the image space of the 2D and 3D images.

The surgical robot 100 is capable of: (1) using registered 2D and/or 3D images for surgical planning, navigation, and guidance in a variety of workflows (e.g., intraoperative 3D, intraoperative 2D, preoperative 3D to 2D, and intraoperative 3D to 2D, etc.); and (2) containing a camera tracking system 200 capable of tracking markers (e.g., NIR retroreflective, NIR LED, visible, etc.). In some cases, as noted herein, a dynamic reference base (DRB) (or patient reference array) 116 is (1) capable of rigidly attaching to the patient anatomy, and (2) contains an array of tracking markers (e.g., NIR retroreflective, NIR LED, visible, etc.).

The XR headsets 150 may be configured to augment a real-world scene with computer generated XR images while worn by personnel in the operating room. The XR headsets 150 may be configured to provide an augmented reality (AR) viewing environment by displaying the computer generated XR images on a see-through display screen that allows light from the real-world scene to pass therethrough for combined viewing by the user. Alternatively, the XR headsets 150 may be configured to provide a virtual reality (VR) viewing environment by preventing or substantially preventing light from the real-world scene from being directly viewed by the user while the user is viewing the computer-generated AR images on a display screen. The XR headsets 150 can be configured to provide both AR and VR viewing environments. Thus, the term XR headset encompasses both or either of an AR headset or a VR headset.

With continuing reference to FIGS. 1-4, the surgical robot 100 may include, for example, one or more robot arms 102, 104, a display 110, an end effector 112, for example, including a guide tube, and an end effector reference element 114 which can include one or more tracking fiducials. A patient reference element (or DRB) 116 (shown in FIG. 1) has a plurality of tracking fiducials and is secured directly to the patient 210. For example, a navigated pelvis DRB marker array may be placed intra-incision or extra-incision with the help of cortical pins drilled into the pelvic bone. In some embodiments, the DRB is oriented to be visible by the tracking camera(s) 204 (e.g., a stereoscopic tracking camera) installed on the camera tracking system 200 and/or the XR headset 150. A reference element 170 is attached to or formed on an instrument, surgical tool, surgical implant device, etc.

The camera tracking system 200 includes tracking cameras 204 which may be spaced apart to provide stereo cameras configured with partially overlapping fields-of-view. The camera tracking system 200 can have any suitable configuration of arm(s) 202 to move, orient, and support the tracking cameras 204 in a desired location, and may contain at least one processor operable to track the location of an individual fiducial and pose of an array of fiducials of a reference element.

As used herein, the term "pose" refers to the location (e.g., along three orthogonal axes, e.g., the x-, y-, and z-axes) and/or the rotation angle (e.g., about the three orthogonal axes) of fiducials (e.g., DRB) relative to another fiducial (e.g., surveillance fiducial) and/or to a defined coordinate system (e.g., camera coordinate system, navigation coordinate system, etc.). A pose may therefore be defined based on only the multidimensional location of the fiducials relative to another fiducial and/or relative to the defined coordinate system, based on only the multidimensional rotational angles of the fiducials relative to the other fiducial and/or to the defined coordinate system, or based on a combination of the multidimensional location and the multidimensional rotational angles. The term "pose" therefore is used to refer to location, rotational angle, or combination thereof of, e.g., an instrument reference element 170, a patient reference element 116, or the like.

The tracking cameras 204 may include, e.g., infrared cameras (e.g., bifocal or stereophotogrammetric cameras) operable to identify, for example, active and passive tracking fiducials for single fiducials (e.g., a surveillance fiducial) and reference elements which can be formed on or attached to the patient 210 (e.g., patient reference element or DRB 116), end effector 112 (e.g., end effector reference element 114), XR headset(s) 150 worn by a surgeon 120 and/or a surgical assistant 126, etc. in a given measurement volume of a camera coordinate system while viewable from the perspective of the tracking cameras 204. The tracking cameras 204 may scan the given measurement volume and detect light that is emitted or reflected from the fiducials in order to identify and determine locations of individual fiducials and poses of the reference elements in three-dimensions. For example, active reference elements may include infrared-emitting fiducials that are activated by an electrical signal (e.g., infrared light emitting diodes (LEDs)), and passive reference elements may include retroreflective fiducials that reflect infrared light (e.g., they reflect incoming IR radiation into the direction of the incoming light), for example, emitted by illuminators on the tracking cameras 204 or other suitable devices.

The XR headsets 150 may each include tracking cameras (e.g., spaced apart stereo cameras) that can track the location of a surveillance fiducial and poses of reference elements within the XR camera headset fields of view (FOVs) 152 and 154, respectively. Accordingly, as illustrated in FIG. 1, the location of the surveillance fiducial and the poses of reference elements on various objects such as, e.g., instrument reference element 170 and patient reference element 116, can be tracked while in the FOVs 152 and 154 of the XR headsets 150 and/or a FOV 212 of the tracking cameras 204.

FIGS. 1 and 2 illustrate a potential configuration for the placement of the camera tracking system 200 and the surgical robot 100 in an operating room environment. Computer assisted navigated robotic surgery can be provided by the surgical robot 100, the camera tracking system 200 controlling the XR headsets 150 and/or other displays 34, 36, and 110 to display surgical procedure navigation information.

The camera tracking system 200 may operate using tracking information and other information provided by multiple XR headsets 150 such as inertial tracking information and optical tracking information (frames of tracking data). The XR headsets 150 operate to display visual information and may play-out audio information to the wearer. This information can be from local sources (e.g., the surgical robot 100), imaging devices 420 (FIG. 4), remote sources (e.g., patient medical image database), and/or other electronic equipment. The camera tracking system 200 may track fiducials in 6 degrees-of-freedom (6 DOF) relative to three axes of a 3D coordinate system and rotational angles about each axis. The XR headsets 150 may also operate to track hand poses and gestures to enable gesture-based interactions with "virtual" buttons and interfaces displayed through the XR headsets 150, and can also interpret hand or finger pointing or gesturing as various defined commands. Additionally, the XR headsets 150 may have a 1-10x magnification digital color camera sensor called a digital loupe. In some embodiments, one or more of the XR headsets 150 are minimalistic XR headsets that display local or remote information but include fewer sensors and are therefore more lightweight.

An "outside-in" machine vision navigation bar 206 supports the tracking cameras 204 and may include a color camera. The machine vision navigation bar generally has a more stable view of the environment because it does not move as often or as quickly as the XR headsets 150 while positioned on wearers' heads. The patient reference element (or, DRB) 116 is generally rigidly attached to the patient 210 with stable pitch and roll relative to gravity. This local rigid patient reference 116 can serve as a common reference for reference frames relative to other tracked elements, such as a reference element 114 on the end effector 112, instrument reference element 170, and reference elements on the XR headsets 150.

In some embodiments, at the end of the end effector 112, instruments are connected to perform operations such as resection, reaming, and implant placement.

The surgical robot 100 may be positioned near or next to patient 210 as shown in FIGS. 1-2. The robot 100 can be positioned at any suitable location near the patient 210 depending on the area of the patient 210 undergoing the surgical procedure. The camera tracking system 200 may be separate from the robot system 100 and positioned at the foot of patient 210. This location allows the tracking camera 200 to have a direct visual line of sight to the surgical area 208, e.g., the hip area (FIG. 2). In the configuration shown in FIG. 1, the surgeon 120 may be positioned across from the robot 100, but is still able to manipulate the end effector 112 and the display 110. A surgical assistant 126 may be positioned across from the surgeon 120 again with access to both the end effector 112 and the display 110. If desired, the locations of the surgeon 120 and the assistant 126 may be reversed. An anesthesiologist 122, nurse, or scrub tech can operate equipment which may be connected to display information from the camera tracking system 200 on a display 34 (FIG. 1).

With respect to the other components of the robot 100, the display 110 can be attached to the surgical robot 100 or in a remote location. The end-effector 112 may be coupled to the robot arm 104 and be controlled by at least one motor. An upper arm 102 may further couple the arm 104 to the column 312 of the robot 100. In some embodiments, end effector 112 includes a guide tube, which is configured to receive and orient a surgical instrument, tool, or implant used to perform a surgical procedure on the patient 210. For example, the end effector 112 is adapted to receive a surgical instrument or a portion thereof, to removably couple to the instrument, and to manipulate the instrument such as by translating and rotating the instrument. In some other embodiments, the end-effector 112 includes a passive structure guiding a saw blade (e.g., sagittal saw) along a defined cutting plane. Although generally shown with a guide tube, it will be appreciated that the end-effector 112 may be replaced with any suitable instrumentation for use in surgery. In some embodiments, end-effector 112 can comprise any known structure for effecting the movement of the surgical instrument in a desired manner.

The surgical robot 100 is operable to control the translation and orientation of the end-effector 112. The robot 100 may move the end-effector 112 under computer control along x-, y-, and z-axes, for example. The end-effector 112 can be configured for selective rotation about one or more of the x-, y-, and z-axes, and a Z Frame axis, such that one or more of the Euler Angles (e.g., roll, pitch, and/or yaw) associated with the end effector 112 can be selectively computer controlled. In some embodiments, selective control of the translation and orientation of end effector 112 and associated surgical instrument can permit performance of medical procedures with significantly improved accuracy compared to conventional robots that utilize, for example, a six-DOF robot arm comprising only rotational axes. For example, the surgical robot 100 may be used to operate on patient 210, and robot arm 104 can be positioned above the body of patient 210, with end-effector 112 selectively angled relative to the z-axis toward the body of patient 210.

In some example embodiments, the XR headset(s) 150 can be controlled to dynamically display an updated graphical indication of the pose of the surgical instrument so that the user, e.g., surgeon 120, can be aware of the pose of the surgical instrument at all times during the procedure.

In some further embodiments, surgical robot 100 can be operable to correct the path of a surgical instrument guided by the robot arm 104 if the surgical instrument strays from the selected, preplanned, or defined trajectory. The surgical robot 100 can be operable to permit stoppage, modification, and/or manual control of the movement of end effector 112 and/or the surgical instrument. Thus, in use, a surgeon 120 or other user can use the surgical robot 100 as part of computer assisted navigated surgery, and has the option to stop, modify, or manually control the autonomous or semi-autonomous movement of the end-effector 112 and/or the surgical instrument.

Fiducials of reference elements can be formed on or connected to robot arms 102 and/or 104, the end effector 112 (e.g., end effector element 114 in FIG. 2), and/or a surgical instrument (e.g., instrument element 170) to enable tracking of poses in a defined coordinate system, e.g., such as in six degrees of freedom (DOF) along three orthogonal axes and rotation about the axes. The reference elements 114, 116, 170 enable each of the marked objects (e.g., the end-effector 112, the patient 210, and the surgical instruments, respectively) to be tracked by the tracking camera 200, and the tracked poses can be used to provide navigated guidance during a surgical procedure and/or to control movement of the surgical robot 100 for guiding the end effector 112 and/or an instrument manipulated by the end effector 112. The instrument manipulated by the end effector 112 may include, e.g., a reamer 124 or an inserter adapted to insert an implant.

Referring to FIG. 3, the surgical robot 100 may include a display 110, upper arm 102, lower arm 104, end effector 112, vertical column 312, casters 314, a table 318, and ring 324 which uses lights to indicate statuses and other information. Cabinet 106 may house electrical components of surgical robot 100 including, but not limited to, a battery, a power distribution module, a platform interface board module, and a computer. The camera tracking system 200 may include a display 36, tracking cameras 204, arm(s) 202 (FIG. 1), a computer housed in cabinet 330, and other components.

In computer assisted navigated surgeries, perpendicular 2D scan slices, such as axial, sagittal, and/or coronal views of patient anatomical structure are displayed to enable user visualization of the patient's anatomy alongside the relative poses of surgical instruments. An XR headset or other display can be controlled to display one or more 2D scan slices of patient anatomy along with a 3D graphical model of anatomy. The 3D graphical model may be generated from a 3D scan of the patient, e.g., by a CT scan device, and/or may be generated based on a baseline model of anatomy which isn't necessarily formed from a scan of the patient.

### Example Surgical System

FIG. 4 illustrates a block diagram of a surgical system 10 that includes a surgical robot 100, a computer platform 400 including, *inter alia,* the camera tracking system 200, imaging device(s) 420, and XR headset(s) 150 which are configured to operate as described herein, according to some embodiments.

The imaging device(s) 420 may include a C-arm imaging device, an O-arm imaging device, other imaging device, and/or a patient image database of 2D and/or 3D images. The XR headset 150 provides a human interface for performing navigated surgical procedures. The XR headset 150 can be configured to provide functionalities, e.g., via the computer platform 400, that include without limitation any one or more of: identification of hand gesture-based commands, and display of XR graphical objects on a display device 438 of the XR headset 150 and/or another display device. The display device 438 may include a video projector, flat panel display, etc. The user may view the XR graphical objects as an overlay anchored to particular real-world objects viewed through a see-through display screen. The XR headset 150 may additionally or alternatively be configured to display on the display device 438 video streams from cameras mounted to one or more XR headsets 150 and other cameras.

Electrical components of the XR headset 150 can include a plurality of cameras 430, a microphone 432, a gesture sensor 434, a pose sensor (e.g., inertial measurement unit (IMU)) 436, the display device 438, and a wireless/wired communication interface 440. The cameras 430 of the XR headset 150 may be visible light capturing cameras, near infrared capturing cameras, or a combination of both.

The cameras 430 may be configured to operate as the gesture sensor 434 by tracking for identification user hand gestures performed within the field-of-view of the camera(s) 430. Alternatively, the gesture sensor 434 may be a proximity sensor and/or a touch sensor that senses hand gestures performed proximately to the gesture sensor 434 and/or senses physical contact, e.g., tapping on the sensor 434 or its enclosure. The pose sensor 436, e.g., IMU, may include a multi-axis accelerometer, a tilt sensor, and/or another sensor that can sense rotation and/or acceleration of the XR headset 150 along one or more defined coordinate axes. Some or all of these electrical components may be contained in a head-worn component enclosure or may be contained in another enclosure configured to be worn elsewhere, such as on the hip or shoulder.

As explained above, the surgical system 10 includes the camera tracking system 200 which may be connected to a computer platform 400 for operational processing and which may provide other operational functionality including a navigation controller 404 and/or an XR headset controller 410. The surgical system 10 may further include the surgical robot 100. The navigation controller 404 can be configured to provide visual navigation guidance to an operator for moving and positioning a surgical tool relative to patient anatomical structure based on a surgical plan, e.g., from a surgical planning function, defining where a surgical procedure is to be performed using the surgical tool on the anatomical structure and based on a pose of the anatomical structure determined by the camera tracking system 200. The navigation controller 404 may be further configured to generate navigation information based on a target pose for a surgical tool, a pose of the anatomical structure, and a pose of the surgical tool and/or an end effector 112 of the surgical robot 100. The navigation information may be displayed through the display device 438 of the XR headset 150 and/or another display device to indicate where the surgical tool and/or the end effector 112 of the surgical robot 100 should be moved to perform a surgical procedure according to a defined surgical plan.

The electrical components of the XR headset 150 can be operatively connected to the electrical components of the computer platform 400 through the wired/wireless interface 440. The electrical components of the XR headset 150 may be operatively connected, e.g., through the computer platform 400 or directly connected, to various imaging devices 420, e.g., the C-arm imaging device, the O-arm imaging device, other imaging device(s), the patient image database, and/or to other medical equipment through the wired/wireless interface 440.

The surgical system 10 may include a XR headset controller 410 that at least partially resides in the XR headset 150, the computer platform 400, and/or another system component connected via wired cables and/or wireless communication links. Various functionality may be provided by software executed by the XR headset controller 410. The XR headset controller 410 is configured to receive information from the camera tracking system 200 and the navigation controller 404, and to generate an XR image based on the information for display on the display device 438.

The XR headset controller 410 can be configured to operationally process frames of tracking data from the cameras 430 (tracking cameras), signals from the microphone 432, and/or information from the pose sensor 436 and the gesture sensor 434, to generate information for display as XR images on the display device 438 and/or for display on other display devices for user viewing. Thus, the XR headset controller 410 as illustrated as a circuit block within the XR headset 150 is to be understood as being operationally connected to other illustrated components of the XR headset 150 but not necessarily residing within a common housing or being otherwise transportable by the user. For example, the XR headset controller 410 may additionally or alternatively reside within the computer platform 400 which, in turn, may reside within the cabinet 330 of the camera tracking system 200, the cabinet 106 of the surgical robot 100, etc.

### Exemplary Patient Registration Workflows

In some embodiments of the present disclosure, the system 10, e.g., computer platform 400, may perform one of a number of available workflows to register a patient to the surgical system 10 prior to surgery. The workflows may further include isolating a target area for the surgical procedure from non-target surgical areas. In one example, the target surgical area may include the acetabulum, and the non-target surgical area may include the femur.

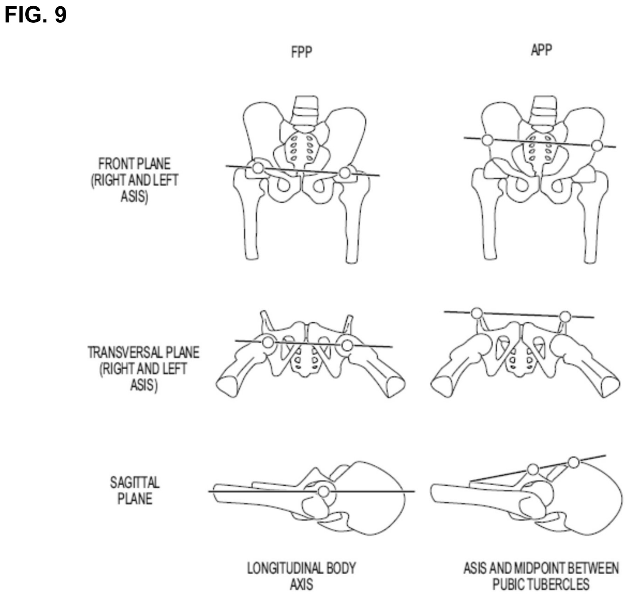

In one embodiment, the workflow may be an imageless workflow in which no pre-operative images are used. Information about the patient anatomy in the operating room (OR) can be obtained by the surgeon measuring key parameters of the patient's bone(s) using the system as described herein. For example, the computer platform 400 of the system 10 operates to identify the locations of landmarks (e.g., points, axes, and/or surfaces) on the bone and to register the locations either concurrently with the identification or thereafter. The locations can be used to define reference plane(s) (e.g., anterior pelvic plane (APP) and/or functional pelvic plane (FPP)) (shown in FIG. 9) which, in turn, are used to plan implants and navigate the robot and surgical instruments for THA surgical procedures.

In some embodiments, the only pre-operative use case associated with the imageless workflow may be the initial patient assessment. The surgeon may assess the patient's mobility and health status with assistance from sensors (e.g., sensors made by Globus Medical which are attached to the leg), physical exercises, and/or clinical surveys to determine if THA is recommended. Gathered data may then be stored and processed by the system 10 before being analyzed by the surgeon to facilitate a final decision. Subsequently, the data may be reused by an application (e.g., surgery planning application by Globus Medical) to establish the most appropriate implant surgical plan.

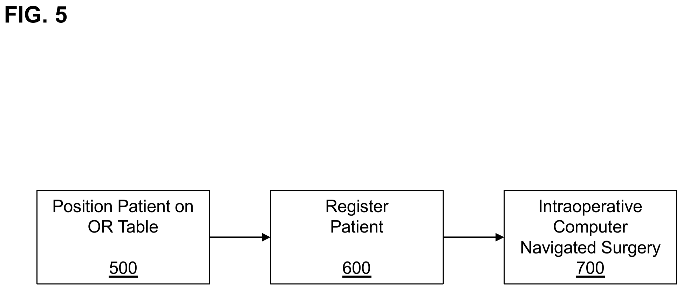

FIG. 5 illustrates a flowchart for an imageless workflow during an intra-operative portion of a THA surgery, in accordance with some embodiments of the present disclosure. In some embodiments, after positioning the patient on the operating room table (process 500), some of the operations discussed above and below may be performed during process 600 to register a patient and before another process 700 for intraoperative computer navigated surgery. In the case of a hip, a pelvis or acetabulum of the patient is registered in the tracking coordinate system of the camera tracking system 200. As shown in FIG. 1, the pelvis or more particularly the acetabulum is registered in the optical coordinate system. In one embodiment, the registration is done in an imageless modality without the use of any medical images such as X-rays or CT images from an imaging device. As noted herein, in other embodiments, registration is performed using one or more pre-operative X-ray images and/or CT images.

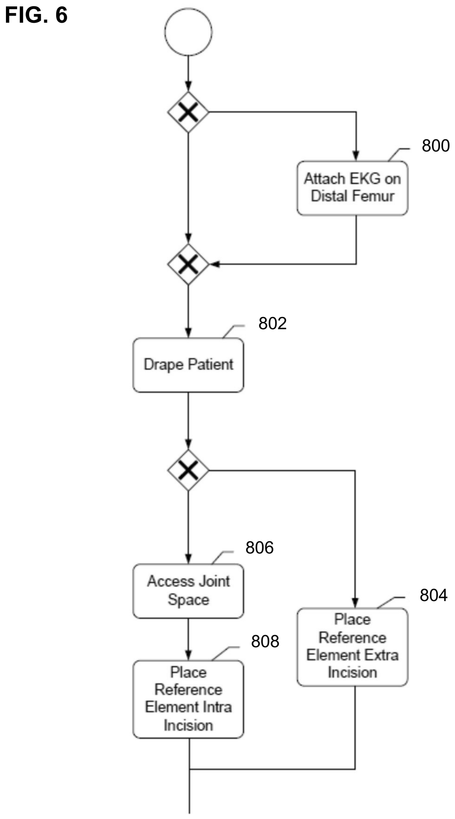

FIG. 6 illustrates a flowchart of a patient preparation process before registration, in accordance with some embodiments of the present disclosure.

The patient preparation process may begin with a patient being positioned in a lateral or supine position on the OR table. The patient's body is prepared for registration. Optionally, in process 800, an EKG/ECG patch electrode is attached on or adjacent a distal end of the patient's femur. The EKG/ECG patch electrode may be placed on the center of the patella or slightly inferior to the center. In some embodiments, the patch location is in line with the anatomic axis of the femur. This patch may be used to acquire the most distal point of the femur under the drape at a later stage. This patch may also be used to track the femur in space (e.g., when the patient's leg is moved during surgery) and may also be used to assist in measuring the patient's leg length. However, in some embodiments, this operation (process 800) is skipped.

In some embodiments, the EKG/ECG patch electrode includes an adhesive patch that is removably attachable to the patient. In some embodiments, the patch may be white, black or dark or have a distinct color to be more visible for tracking by the tracking camera. In other embodiments, the patch and patch electrodes are not visible by the tracking camera as they are under a drape. The patch geometry (like a nipple) will help the surgeon to always touch a single point on or adjacent the distal part of the femur (anterior patella region) with a navigated stylus/instrument which is trackable by the tracking camera. This ensures that the surgeon always collects the same point to measure the leg length or medio-lateral offset, or register patient anatomy data points for patient registration.

In process 802, the patient body is draped. Then, depending on the surgeon's technique, the navigated pelvis DRB, e.g., DRB 116 (FIG. 2), is placed intra-incision (processes 806-808) or extra-incision (process 804) with the help of cortical pins drilled into the pelvic bone. In some embodiments, the DRB 116 is oriented to be visible to the tracking camera(s), e.g., stereoscopic tracking cameras 204 installed on the camera tracking system 200 (FIG. 1) or the XR headset 150 (FIG. 1). In one embodiment, the operation to place the DRB intra-incision includes using the system 10 to track and navigate access to the joint space (process 806) and placing the reference element intra-incision. In an alternative, more preferred embodiment, the reference element 116 is placed extra-incision (process 804) without tracking the DRB. Once the DRB is placed, the surgeon can register the anatomy, plan implants and perform the surgical operation.

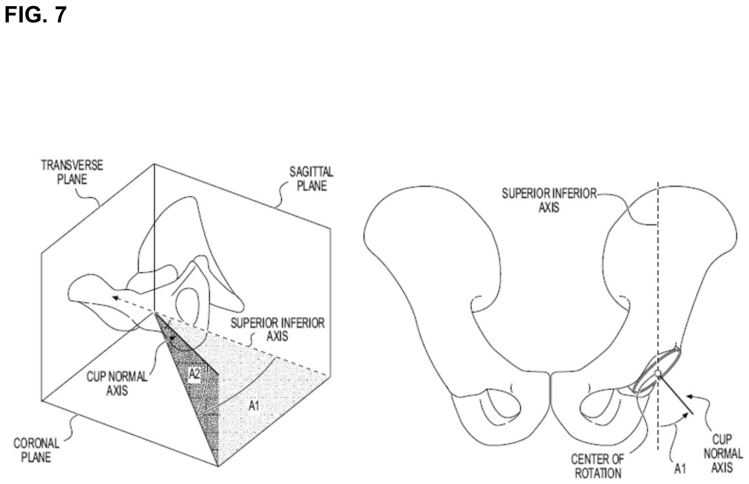

After the reference element, e.g. DRB 116 has been placed intra-incision or extra-incision, data points and axes can be collected on the patient anatomy with the assistance of navigated instruments and using the pelvis DRB coordinate system as a spatial reference. In addition to this, two pelvic reference planes can be established to plan placement of implants by measuring angular deviations such as inclination and version of the acetabular cup implant as shown in FIGS. 7-8.

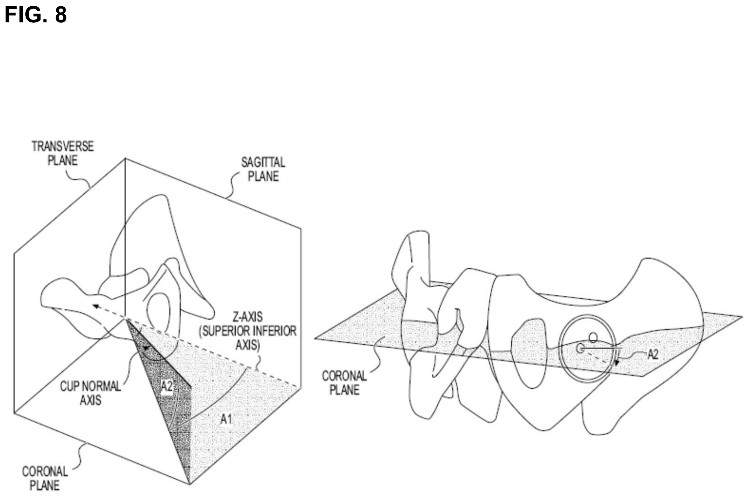

FIG. 7 illustrates a radiographic inclination angle measured in the coronal plane of the patient, in accordance with some embodiments of the present disclosure. In some embodiments, the surgeon may use a navigated instrument to palpate or paint the surface of the acetabular cavity of the patient to determine a center of rotation of the acetabulum. FIG. 8 illustrates a radiographic version angle measured relative to the coronal plane of the patient, in accordance with some embodiments of the present disclosure. The two pelvic reference planes (or coronal or frontal planes), the anterior pelvic plane (APP) and functional pelvic plane (FPP), are determined or defined using different landmarks and axes as shown on FIG. 9 and described in further detail below.

It is to be understood herein that although the user interfaces and associated operations are described as being performed in a certain sequence, they may be performed in other sequences while still being within disclosed embodiments. Moreover, it is not necessary that all of the user interfaces and/or described operations be performed. Instead, fewer operations may be performed while still being within disclosed embodiments. Further, additional registration approaches can include image-based and imageless workflows. Combinations of these registration approaches are also possible in keeping with the various disclosed embodiments.

During a patient registration procedure, landmarks used to register patient anatomy can be extracted using either single point palpation collection or surface painting, resulting in a point cloud of locations. FIG. 9 illustrates different views of landmarks and axes for registration of the FPP and APP of a patient, in accordance with some embodiments of the present disclosure. The landmarks and axes used to register the APP and FPP planes are described in more detail in US Patent Application No. 18/430,077 (filed February 01, 2024), previously incorporated by reference herein.

US Patent Application No. 18/430,077 further discloses processes for registration of a pelvic acetabulum of a patient (including painting the acetabular cavity), in accordance with various embodiments of the present disclosure. For example, to define the APP and FPP origins, the pelvic acetabular center of rotation can be determined after removing the femoral head of the patient from the acetabular cavity. The acetabular cavity may be made accessible by cutting the femoral neck and by removing the femoral head from the acetabular cavity. In some embodiments, a cork screw instrument may be used to remove the femoral head from the acetabular cavity.

The surface of the acetabular cavity can then be painted using the navigated instrument (e.g., a stylus). For example, the surgeon may use the navigated instrument (e.g., stylus) to palpate the surface of the acetabular cavity, as the tracking camera measures the position of a ball on the end of the stylus in a continuous way. This process provides a cloud of points for the measured positions (locations). At the same time, the tracking camera may also monitor and track the pose of the patient DRB 116 attached to the pelvis such that the pose of the stylus can be tracked relative to the pose of the patient DRB. Alternatively, the surgeon may subsequently measure a predefined number or percentage of points by palpating them one-by-one. Based on these points and the tracking data of the stylus and patient DRB 116, the center of rotation of the pelvic acetabular cavity is determined. Additionally, based on these points, the surface of the acetabular cavity may be registered in the system and/or a 3D model may be generated or modified based on these points. Next, the acetabular cavity shape can be recreated (e.g., in a 3D model) by the system based on the measured cloud of points and using other algorithms, e.g., for outlier removals and surface fitting.

While certain imageless approaches are described herein and in US Patent Application No. 18/430,077 (filed February 01, 2024), previously incorporated by reference herein, other example methods of performing imageless and image-based registration of the pelvis to the tracking coordinate system of the tracking system (e.g. optical coordinate system). These methods may also be used to, e.g., determine a native center of rotation of the acetabulum, derive or define an FPP, and derive or define an APP. Registration may allow a navigation system or robotic system to track any navigated instrument or end effector 112 or any tool attached to the end effector 112 relative to the pelvis as tracked by a patient dynamic reference base 116 attached to the pelvis. Various registration methods described herein can be combined in keeping with various disclosed embodiments.

For example, in one imageless method, an APP is derived by either touching various known points (e.g., left and right anterior superior iliac spine (ASIS) and pubic symphysis) with a navigated instrument, or by a physician lining up a plane or axis defined by the navigated instrument along or parallel to the APP. With the center of rotation and APP determined, the system (either a navigation system or a combined navigation and robot system 100) has sufficient information to register the acetabulum in the coordinate system (e.g., optical coordinate system) of the camera tracking system 200. In both of the above-noted example methods, the tracking system may be constantly monitoring and tracking the pose of the patient DRB 116 attached to the pelvis while also tracking the navigated instrument (e.g., stylus) such that the pose of the instrument can be tracked relative to the pose of the patient DRB, at least for purposes of registering the pelvis relative to the patient DRB 116 in the tracking coordinate system of the camera tracking system 200.

Some exemplary image-based examples include the use of pre-operative CT images. In one such exemplary image-based approach to patient registration, pre-operative CT and intra-operative fluoroscopy images are merged, the non-target surgical area is excluded, and the location of the target surgical area is registered based on the merged CT image and fluoroscopy image, as described in Patent Application No. 18/743,388 (filed June 14, 2024). In another exemplary image-based approach to patient registration, pre-operative CT and intra-operative point cloud data acquired via a navigated instrument are merged, and the location of the target surgical area is registered based on the merged CT image and point cloud data, as described in Patent Application No. 18/743,615 (filed June 14, 2024).

Other exemplary image-based registration approaches can be performed without first obtaining pre-operative CT images. In one such exemplary approach to patient registration, intra-operative fluoroscopy images are obtained, and an APP and FPP are identified. The FPP images and APP images are merged, excluding the non-target surgical area, and the location of the target surgical area is registered based on the merged APP and FPP fluoroscopy images, as described in Patent Application No. 18/743,647 (filed June 14, 2024).

In a further exemplary image-based approach to patient registration, intra-operative fluoroscopy images are obtained, and intra-operative point cloud data is acquired using a navigated instrument. An FPP is identified in the intra-operative fluoroscopy images, and inputs from a navigated instrument about a location of the target surgical area are obtained. A set of landmarks relative to the identified FPP are verified using the inputs from the navigated instrument, and the location of the target surgical area is registered based on the identified FPP images and inputs from the navigated instrument, as described in Patent Application No. 18/743,685 (filed June 14, 2024).

Computer-assisted navigation systems provide surgeons with computerized visualization of how a surgical instrument or other device that is posed relative to a patient correlates to a pose relative to medical images of the patient's anatomy, and how those poses correlate to a pre-operative surgical plan.

### Acetabular Center of Rotation (CoR) Determination

As described herein, patient registration methods match patient anatomy with digital representations of the corresponding bone. In the context of THA procedures, relevant anatomical features include the pelvis and femur, and features thereof. The acetabular center of rotation (CoR) is an important reference point for use in planning and performing THA procedures. Provided herein are several methods of registering the acetabular CoR on images or directly on patient anatomy using a dedicated tracking instrument. The registration methods described herein may be used in the framework of an imageless registration approach, a 2D image approach (e.g., intra-operative fluoroscopy) (US Patent Application No. 18/743,647), a 3D image approach (pre-operative CT scan and intra-operative point cloud acquisition) (US Patent Application No. 18/743,615), and a 2D/3D image approach (pre-operative CT and intra-operative fluoroscopy) (US Patent Application No. 18/743,388). Each of the foregoing referenced patent applications was filed June 14, 2024, and was previously incorporated by reference.

The acetabular CoR registration methods described herein provide a number of advantages. For example, the methods described herein facilitate the simplified and flexible determination of the acetabular center of rotation on patient bone surface, 2D images, or 3D images. The present methods also enable numerical determination of the acetabular CoR using appropriate algorithms, which reduces errors of acquisition and improves the bone modeling and corresponding registration. Additionally, the exact shape of the acetabular cavity can be acquired and further integrated into the process of bone modeling. Acetabular CoR based on Surface Palpation

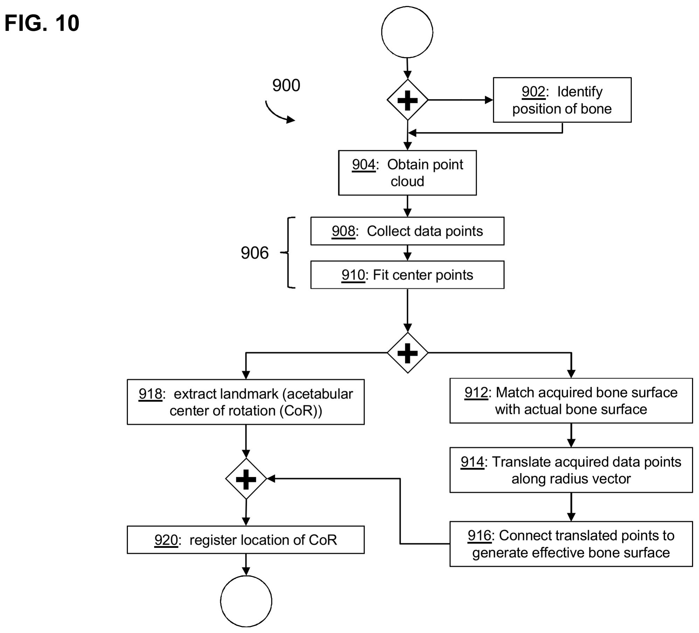

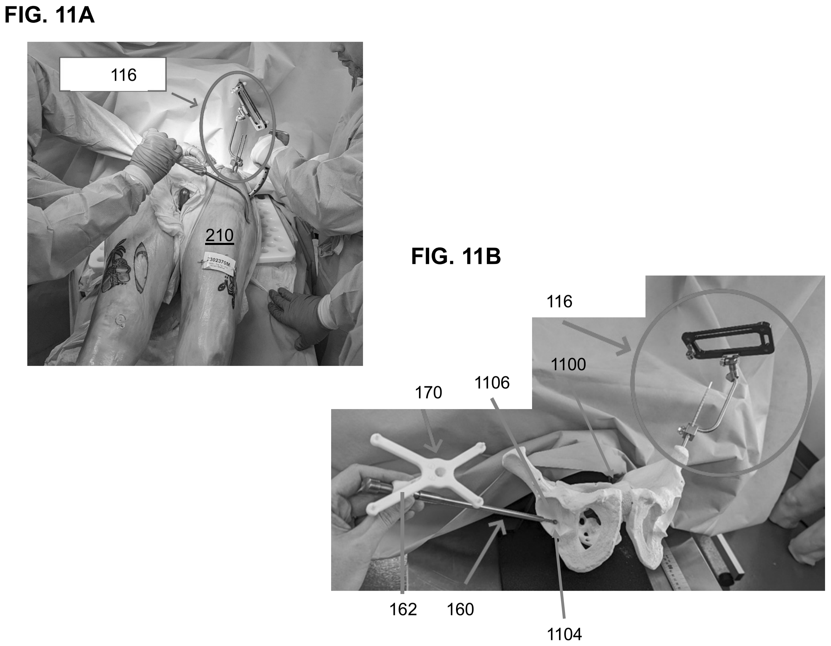

Turning to the figures, in various embodiments, a workflow 900 is provided in FIG. 10, and aspects are illustrated in FIGS. 11-15, for determining and registering a landmark such as, e.g., an acetabular CoR, by palpating a bony surface of an anatomical feature such as the acetabulum with a navigated instrument to acquire a cloud of data points detected and captured on the surface of the bone. This point cloud may be used to derive and register the landmark, e.g., the acetabular CoR, as described herein. The processes of workflow 900 may be performed using the system 10 as described above, including, e.g., computer platform 400, camera tracking system 200, surgical robot 100, and other components depicted in FIGS. 1-4 and described herein above.

With reference to FIG. 10, as noted above, workflow 900 provides processes for determining and registering a landmark such as, e.g., an acetabular CoR, for use in computer assisted navigation during a THA surgery. The workflow may be performed at least in part on a computer platform such as computer platform 400 (FIG. 4), which may include a processor and a memory, operative to carry out certain processes of the workflow described herein.

A first, optional process 902 includes rigidly affixing a reference element such as DRB 116 to the pelvis 1100 of the patient 210, as illustrated in FIGS. 11A-11B. The DRB 116 may include a tracking array including tracking markers or fiducials which are detectable by the camera tracking system 200 (FIGS. 1-4) as described herein. The array may be affixed to the pelvis 1100 with a fixation structure, e.g., screw pins, and oriented such that it is visible to the camera tracking system 200. Once rigidly affixed, the DRB 116 thus allows tracking of the pelvis 1100 by the camera tracking system 200, as well as tracking of the pose(s) of navigated instruments relative to the DRB 116. The position and orientation of the DRB 116 remain rigidly fixed with respect to the bone throughout the procedure. Using the camera tracking system 200 and the computer platform 400, the position of the pelvis 1100 relative to the DRB 116 is identified. This may include measuring natural landmarks such as, e.g., pelvic Anterior Superior Iliac Spine (ASIS) points, acetabular rim points, a femoral distal mechanical axis point, and so on. These measurements may be captured by acquiring their localization on the bones, or acquiring specific points that are further used to calculate axes, reference planes, center of rotation positions, and so on.

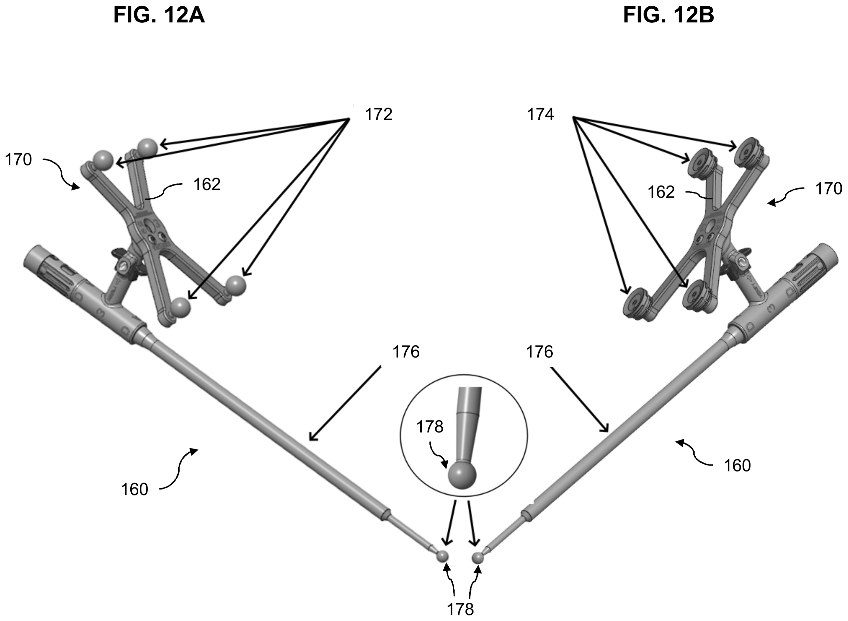

Process 904 includes intra-operatively obtaining a point cloud of a surface 1106 of an acetabulum 1104 of a patient 210 by palpating the surface 1106 with a navigated instrument 160. The point cloud includes a plurality of data points that are detected and captured on the surface 1106 of the acetabulum 1104 by the navigated instrument 160. The navigated instrument 160, shown in detail in FIGS. 12A-12B, may include a pointer or stylus 176 including a ball tip 178 disposed at a distal end thereof, and an instrument reference element 170 affixed to the stylus 176, e.g., near the proximal end of the stylus 176. The instrument reference element 170 may include a tracking array 162 having a plurality of tracking fiducials 172, 174 affixed thereto. In certain embodiments, the tracking fiducials may include sphere markers 172 (FIG. 12A), while in other embodiments, disk markers 174 may be used (FIG. 12B).

In certain embodiments, the navigated instrument 160 may be used in a freehand manner by a user, e.g., surgeon 120, to obtain the point cloud according to instructions and/or navigational guidance provided by the navigation controller 404. In other embodiments, the navigated instrument 160 may be coupled to the end effector 112, which may be in turn coupled to the robotic arm of a surgical robot such as robot 100. In such an embodiment, the movement of the navigated instrument, including translation, rotation, orientation, etc., may be controlled by navigation controller 404.

In certain embodiments, the point cloud may be obtained by sweeping or painting the surface 1106 of the bone, e.g., the acetabulum 1104, with the ball tip 178 of the stylus 176. As the ball tip 178 passes over the features of the surface 1106, the camera tracking system 200 captures the pose of the stylus 176 relative to, e.g., the DRB 116 in a continuous manner. This facilitates the generation and acquisition of a cloud of data points representing the surface 1106. The location of each point is ascertainable relative to the stylus 176 and the ball tip 178 thereof, and DRB 116 by the camera tracking system 200 and computer platform 400.

In other embodiments, the user or surgeon 120 may obtain the point cloud by individually contacting a plurality of points on the surface 1106 of the acetabulum 1104 with the ball tip 178 of the stylus 176. The camera tracking system 200 captures the pose of the stylus 176 as it individually contacts each of the points to be captured. The camera tracking system 200 may thus capture the location of the ball tip 178 at the time of data acquisition, and store that location as a data point. The acquisition of each data point may be automatic or it may be user-actuated. For example, the user 120 may instruct the computer platform 400 to capture each data point, e.g., using any of various functionalities provided by the system 10 such as, e.g., a foot pedal, a control on the stylus 176, a functionality provided by XR headset 150, or another device. This process may be repeated iteratively to obtain the point cloud.

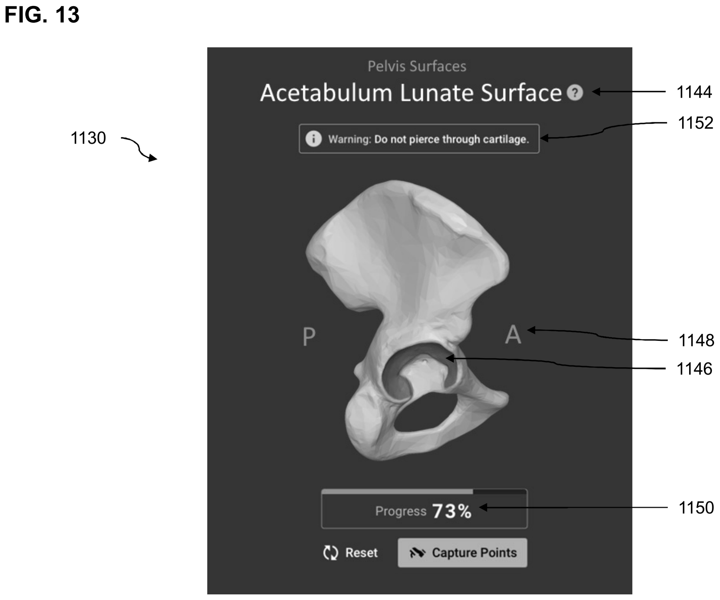

With reference to FIG. 13, the computer platform 400 may include a display such as, e.g., XR headset 150 or display 438, which may display navigational guidance to the user or surgeon 120. This guidance may be presented via a user interface 1130, and may be adapted to assist the surgeon 120 in obtaining the point cloud. The guidance provided to the user may include, e.g., instructions provided on the user interface 1130 to guide or assist the user in acquiring the correct and/or complete surface area, a textual identification of the particular surface being acquired 1144, a graphical representation of the surface being acquired 1146, references providing context for the graphic representation such as, e.g., indications of anterior ("A") and posterior ("P") directions 1148, an updated indication of an extent of completeness of the point cloud acquisition 1150, which may be expressed, e.g., as a percentage of points collected, warnings that may be relevant to the present acquisition 1152 process, and other types of guidance.

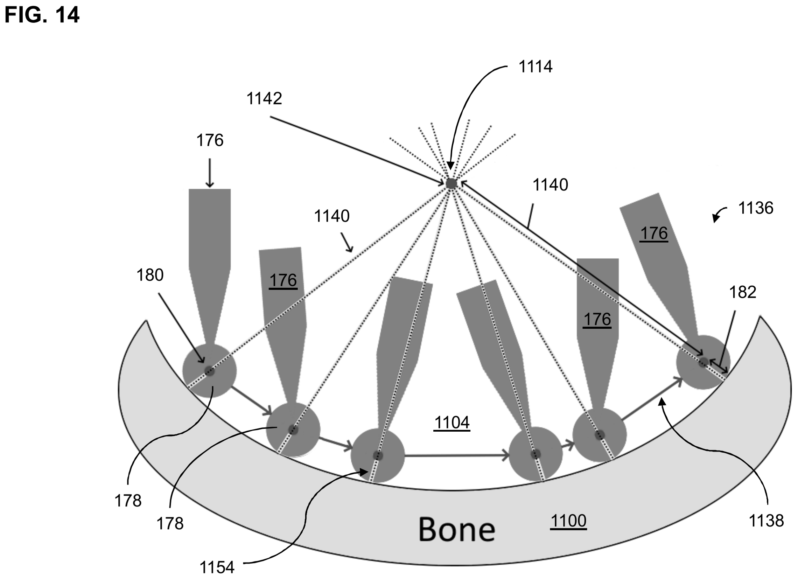

In embodiments in which a stylus 176 having a ball tip 178 is used to obtain the point cloud, each data point in the plurality of data points making up the point cloud corresponds to the location of the center 180 of the ball tip 178 during the acquisition of the respective data point. As a result of the regular geometry of the spherical ball tip 178, the acquired surface is capable of being defined independently of the orientation of the stylus 176 relative to the surface of the acetabulum 1104 at the time of capturing of each data point in the plurality of data points. This is illustrated in FIG. 14, which illustrates the collection of six different data points over time using a stylus 176. The angle of the stylus 176 need not remain constant relative to the surface 1106 of the acetabulum 1104 during point cloud acquisition.

Referring back to workflow 900 of FIG. 10, process 906 includes creating an acquired surface of the acetabulum 1104 based on the point cloud. Process 906 may include sub-processes, e.g., 908 through 910. At process 908, the computing device 400 collects the plurality of data points. Each collected data point represents the location of the center point 180 of the ball tip 178 at the acquisition of the respective data point. Six data points, collected in series over a period of time, are shown in FIG. 14 in a simplified example.

Process 910 includes using a sphere fitting algorithm to fit together the data points collected in process 908. The sphere fitting algorithm may be based, e.g., on least squares mathematical methods, Singular Value Decompositions (SVD), or Random Sample Consensus (RANDSAC) algorithms. The output of the sphere fitting algorithm yields a fitting sphere 1136. The surface 1138 of the fitting sphere 1136 is offset from the surface 1106 of the acetabulum 1104 by a distance corresponding to a radius 182 of the ball tip 178. The surface 1138 of the fitting sphere 1136 corresponds to the acquired surface of the acetabulum 1104.

After the acquired surface of the acetabulum 1104 is created in process 906, the acquired surface may be used to extract one or more landmarks such as, e.g., the acetabular CoR (process 918) and/or to be further transformed via processes 912, 914, and 916 to generate the effective bone surface. The effective bone surface corresponds to a 3D digital model of the surface 1106 of the acetabulum 1104.

Process 912 includes matching the acquired surface to the surface of the acetabulum 1104 to yield the effective bone surface. The matching may include calculating, for each data point, the local ball tip sphere radius vector 1154 to the surface. This ball tip radius vector 1154 is aligned with the fitting sphere radius 1140. At process 914, after all radius vectors are established, each data point is then translated along the ball tip sphere radius vector 1154 by a distance corresponding to the radius 182 of the ball tip 178 in a direction away from the instrument reference element 170 on the stylus 176. Process 916 includes connecting the translated points together to generate or create the effective bone surface. The effective bone surface may then be used in the generation of a 3D model of the bone, and in the further planning and execution of the THA procedure.

Process 918 includes extracting an acetabular center of rotation (CoR) 1114 from the acquired surface. The acetabular CoR 1114 corresponds to a center of the fitting sphere 1136, and can accordingly be algorithmically derived from the acquired surface. In certain embodiments, additional processes may be performed to extract additional landmarks from the acquired surface, e.g., the fitting sphere 1136. Measurements may also be extracted such as, e.g., an acetabular diameter. The location of the patient's acetabular CoR 1114 may be registered to the system in process 920.

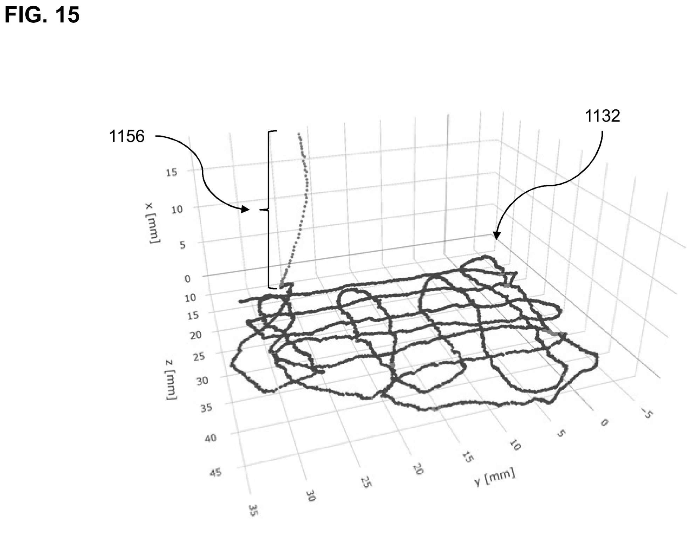

In certain embodiments, the computer platform 400 may further carry out processes for detection and removal of outlier data points. With reference to FIG. 15, during acquisition of the point cloud 1132, data points may be inadvertently collected when the ball tip 178 (FIGS. 12A-12B) is not in contact with the bone surface. This can lead to acquisition and accumulation of numerous non-relevant points in the point cloud. These non-relevant points may be identified using an algorithm that processes the acquired points and identifies and remove outliers from the relevant surface. Thus, the acquired surface may be defined by a majority of the acquired points, e.g., the data points which correspond to a contact of the ball tip 178 of the stylus 176 with the bone, but in many cases the acquired surface may be defined by fewer than all acquired points. In the example of FIG. 15, a set of points is acquired on a resected surface of bone, which should be substantially planar. However, points 1156 were acquired when the surgeon was removing the stylus ball tip 178 from the surface 1106. They are accordingly identified as outliers by the algorithm, and omitted from the creation of the acquired surface.

In various embodiments, the size and shape of the tip of the stylus 176 may vary, impacting various aspects of the processes described herein, as well as the ease with which the tip moves across the bone. A spherical tip, such as ball tip 178, provides ease of gliding along the surface of the bone. However, the use of a spherical-tipped stylus results in certain additional processes to account for the radius of the tip when using the collected data, e.g., to generate the effective bone surface as described herein. A relatively larger ball tip radius 182 is associated with relatively improved glide capabilities, but also several drawbacks. A relatively larger radius 182 contributes to the acquisition of a relatively smoother painted surface as compared to the surface acquired by a ball tip stylus having a smaller tip radius. A smoother surface may result in the potential loss of certain bone asperities that can be of interest for landmark extraction, e.g., detection of the acetabular fossa deepest point from the acetabulum spherical surface. Additionally, for single point acquisitions (i.e., points that are not part of a painted surface), the error induced by the ball tip radius 182 is larger to compensate when a larger tip radius is used. Accordingly, a more accurate ball tip radius vector orientation is used for the translation of the acquired ball tip center to the actual surface of the bone in such embodiments. However, a relatively larger ball tip radius 182 offers convenience for the registration via single point acquisition (i.e., no surface painting) of both the acetabular center of rotation and final shell diameter matching the patient anatomy.

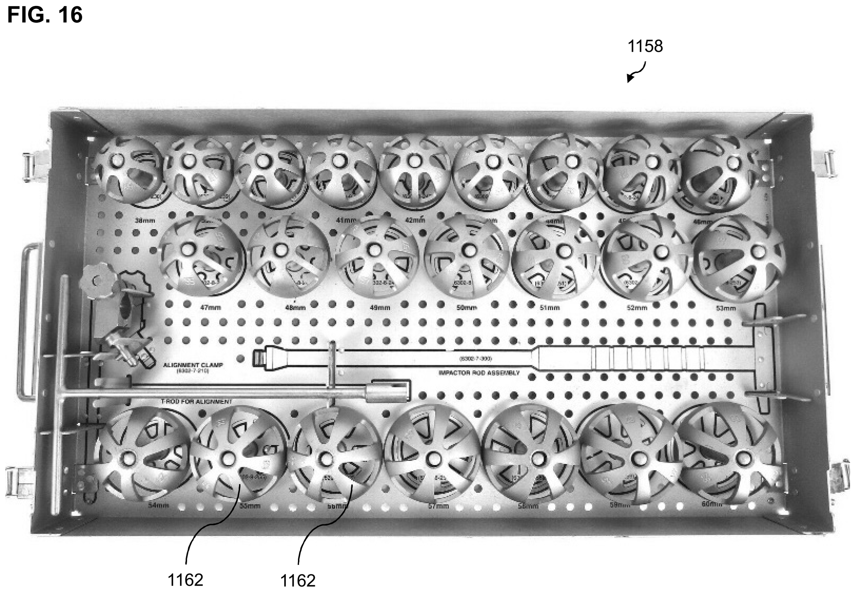

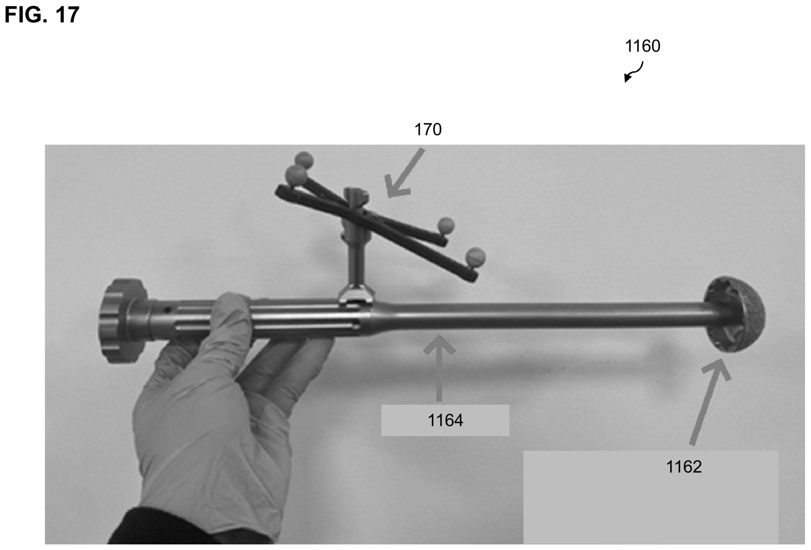

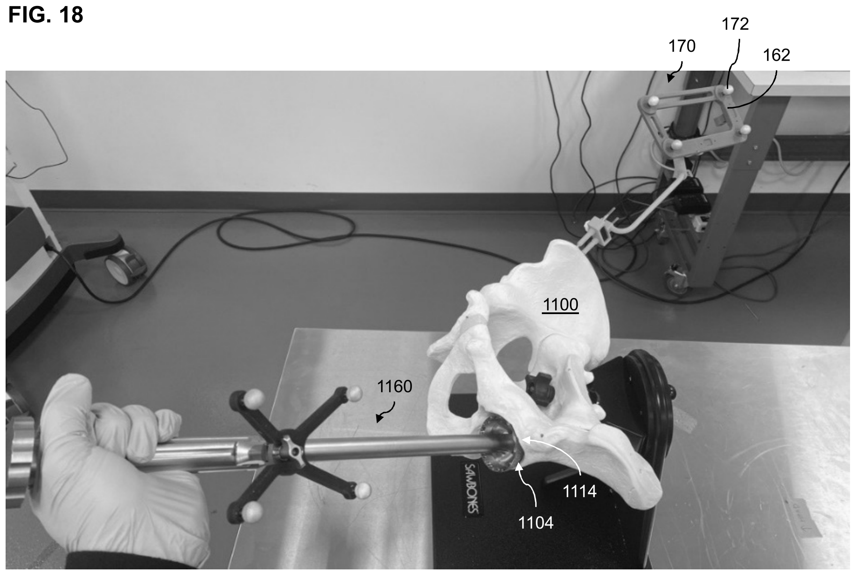

Turning next to FIGS. 16-18, another embodiment of a process for determining the acetabular CoR is described herein. According to this embodiment, a set 1158 of trial acetabular shell components (i.e., hemispherical trial shell implants with different diameters, see FIG. 16) is used to determine the diameter of the patient's acetabulum by iteratively trying different trial shell sizes until the correct size is identified. Once the appropriate size trial shell 1162 has been identified, the user can place the trial implant 1162 into the patient acetabulum using a navigated acetabular shell inserter 1160 (see FIG. 17) that includes the inserter 1164, the trial shell 1162 coupled to a distal end thereof, and a reference element 170 as described elsewhere herein. With the help of the camera tracking system 200, the user can use the navigated inserter 1160 to perform a single point acquisition that will determine the acetabular center of rotation 1114 referenced into the pelvis DRB 116 and localized at the extremity of the navigated acetabular shell inserter 1160 (see FIG. 18).

### Acetabular CoR based on 2D imaging

According to certain other embodiments of the disclosure, the registration of the acetabular CoR can be accomplished using 2D images such as, e.g., x-ray or fluoroscopy images.

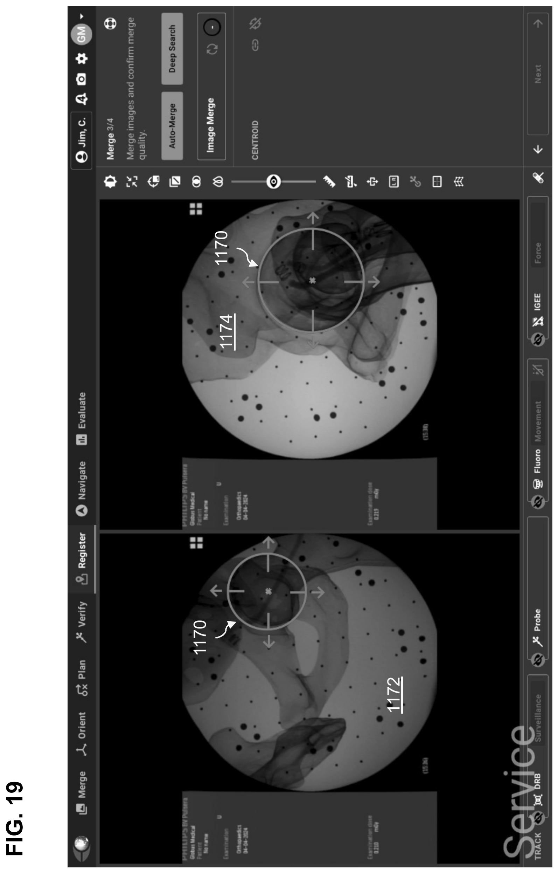

In one embodiment, the acetabular CoR may be determined by the user, e.g., surgeon, with the aid of the computer platform 400 according to a manual process. In one embodiment, the 2D images may be displayed on a display such as, e.g., display 438 (FIG. 3). The user may view the 2D images on the display, and may utilize a registration workflow in which a center point reticle 1170 is capable of manipulation and placement by the user on each of two 2D images, 1172, 1174, as shown in FIG. 19. The view angle between the two images 1172, 1174 may be approximately 90° to reflect the two orthogonal view directions in the 3D space. For example, the two 2D images may be an anterior-posterior (AP) image and a lateral image of the same anatomy, respectively. As described in US Patent Application Nos. 18/743,388 and 18/743,647, both filed June 14, 2024, and both previously incorporated by reference, the position of each of the 2D images is known with respect to the patient pelvis reference element, e.g., DRB 116, using a tracked fluoro fixture device mounted on the imaging device 420 (FIG. 4), e.g., a C-arm. As a result, the exact position on the 2D images of the manually placed center point reticle 1170 is known with respect to the tracked patient anatomy.

In another embodiment, the acetabular CoR may be automatically determined by the system 10 using 2D images such as, e.g., x-ray or fluoroscopy images. According to this embodiment, the computer platform 400 may include software stored in a memory which, which executed by a processor, uses a trained neural network and a machine learning algorithm to automatically extract the position of the acetabular CoR from the 2D images. To facilitate this method of determining the acetabular CoR, an initial dataset of fluoroscopy images with manually annotated acetabular centers of rotation are provided to the algorithm to train the neural network prior to deployment for automatic acetabular CoR determination.

### Acetabular CoR based on 3D imaging

According to certain other embodiments of the disclosure, the registration of the acetabular CoR can be accomplished using 3D images such as, e.g., MRI or CT scans.

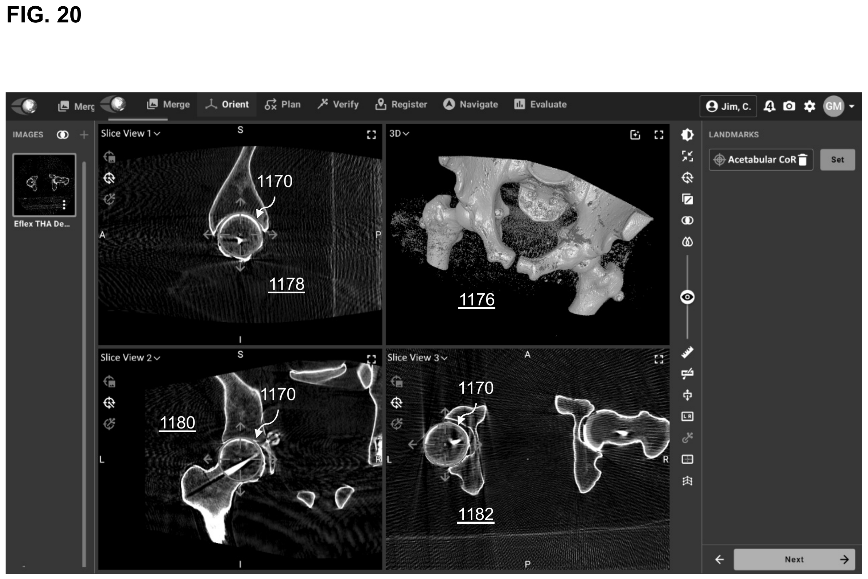

In one embodiment, the acetabular CoR may be manually determined by the user, e.g., surgeon, with the aid of the computer platform 400 according to a manual process. In one embodiment, the 3D scans may be displayed on a display such as, e.g., display 438 (FIG. 3), and segmented into slice views. The user may view the segmented 3D images on the display, and may utilize a registration workflow in which a center point reticle 1170 is placed on each of three slice views, as shown in FIG. 20. The reticles 1170 are placed so as to identify the acetabular center of rotation, based on the pelvis bone model 1176 and the three slice views 1178, 1180, 1182 of the 3D scan. After 3D model registration as described in US Patent Application Nos. 18/743,388 and 18/743,615, both filed June 14, 2024 and both previously incorporated by reference, the location of the 3D-image based acetabular center of rotation is known on the anatomy of the patient.

In another embodiment, the acetabular CoR may be automatically determined by the system 10 using 3D images such as, e.g., MRI or CT scans. According to this embodiment, the computer platform 400 may include software stored in a memory which, which executed by a processor, uses a trained neural network and a machine learning algorithm to automatically extract the position of the acetabular center of rotation from the segmented 3D images. To facilitate this method of determining the acetabular CoR, an initial dataset of 3D images, e.g., CT scans, with manually annotated acetabular centers of rotation are provided to the algorithm to train the neural network prior to deployment for automatic acetabular CoR determination.

### Functional Computed Tomography (CT) Alignment

Weight bearing images are frequently used for patient assessment and planning purposes prior to a THA procedure. For example, weigh-bearing 2D images including lateral x-rays taken in a seated position, lateral x-rays taken in a standing position, and anterior-posterior (AP) x-rays taken in a standing position may be useful for the planning of the position of an acetabular shell in a THA procedure. Weight-bearing 2D images are useful for their ability to represent the position in which the patient's pelvis will naturally be positioned, allowing the surgeon to account for the patient's functional alignment when planning the implant position. However, such 2D images are limited in the level of detail that can be returned.

3D images such as CT scans are typically acquired with the patient in a non-weight bearing, e.g., supine position. Accordingly, other factors such as the patient's spinal stiffness, any spinal deformities, and pelvic mobility, which may contribute to the patient's functional alignment, are not reflected in the positioning and alignment shown in the resulting 3D image. 3D images such as CT scans remain a useful tool because they provide a level of detail that 2D images cannot.

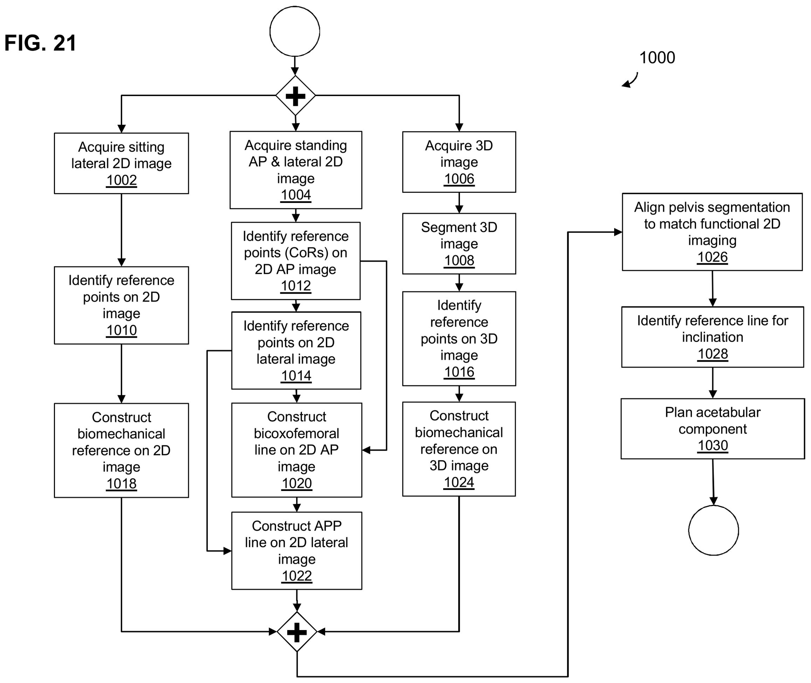

Turning to FIGS. 21-28, in various embodiments, a workflow 1000 is provided for aligning a 3D image such as, e.g., a CT scan, such that the 3D image represents the functional alignment of a patient. The resulting functionally aligned 3D image may then be used to plan the placement of an acetabular shell during a THA procedure including, e.g., the inclination and version angles of the shell. The processes of workflow 1000 may be performed in whole or in part by the system 10, including, e.g., computer platform 400 and imaging device(s) 420, depicted in FIG. 4 and described herein above.

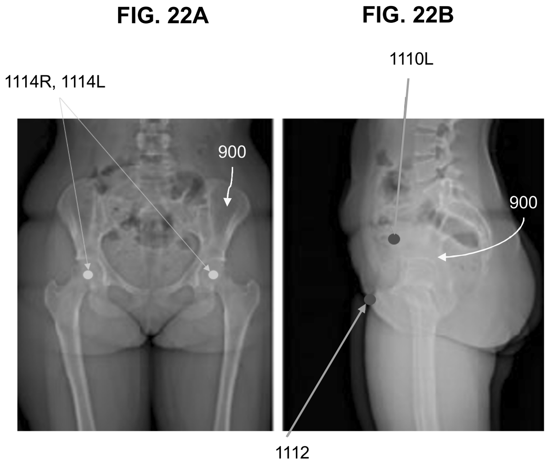

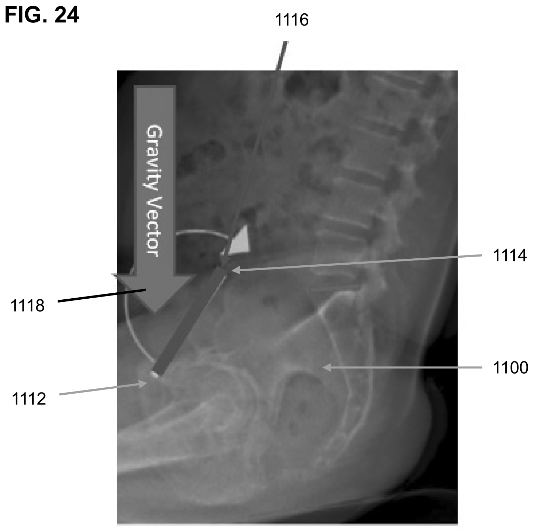

FIG. 21 provides a work flow diagram depicting a process 1000 according to one embodiment of the disclosure. As shown in FIG. 21, at processes 1002, 1004, and 1006, the respective patient images are acquired, e.g., by imaging the patient or by retrieving a previously acquired image of the patient from, e.g., Picture Archiving and Communication System (PACS). In certain embodiments, the images may be acquired using imaging device(s) 420 of system 10 (FIG. 4), which may include imaging devices as well as an image database. In particular, process 1002 includes acquiring a weight-bearing 2D lateral image of the patient's pelvis in a seated position (see, e.g., FIG. 24). Process 1004 includes acquiring weight-bearing 2D anterior-posterior (AP) and lateral images of the patient's pelvis in a standing position (see, e.g., FIGS. 22A and 22B, respectively). As the 2D images acquired in processes 1002 and 1004 (e.g., FIGS. 22A, 22B, 24, 25A, and 25B) are acquired in weight-bearing positions, these images represent the native functional alignment of the pelvis. The 2D images may particularly be x-ray images.

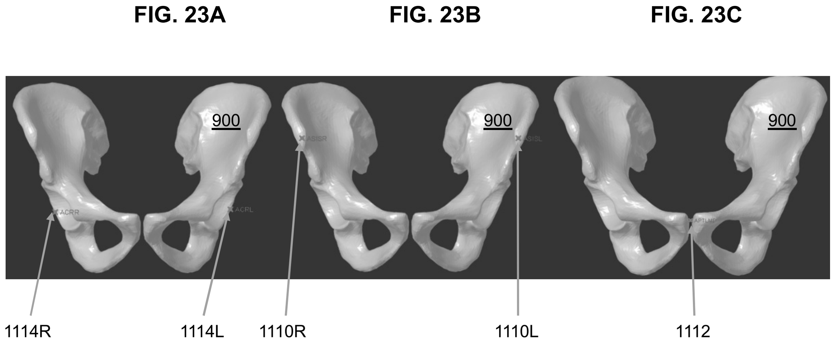

Process 1006 includes acquiring a 3D image such as, e.g., a CT scan of the patient's pelvis, which may depict the patient in a non-weight bearing position such as a supine position (see, e.g., FIGS. 23A-23C).

Process 1008 includes segmenting the 3D image, e.g. the CT scan, into a plurality of segments collectively making up the segmented 3D image. Each segment in the plurality of segments may correspond to a 2D slice of the 3D volume of the 3D image, e.g., CT scan. Collectively, the segments of the 3D image may be referred to as a segmented 3D image. The segmenting process 1008 may be performed using computer platform 400 (FIG. 4). The 2D and 3D images may be displayed on a display of system 10 such as, e.g., display device 438 (FIG. 4), display 36 (FIG. 3), or another display.

Processes 1010, 1012, and 1014 include identifying a plurality of reference points on each of the 2D images depicting the functional alignment of the patient's pelvis in the respective weight-bearing positions. In particular, at process 1010, reference points including, e.g., the anterior superior iliac crests 1110L and 1110R and pubic symphysis 1112, are identified on the lateral sitting 2D image (not shown). Process 1012 includes identifying reference points including, e.g., the left and right acetabular centers of rotation (CoRs) 1114L and 1114R, respectively, on the standing AP 2D image (FIG. 22A). Process 1014 includes identifying reference points including, e.g., the left and right anterior superior iliac crests 1110L and 1110R and pubic symphysis 1112, on the standing lateral 2D image(s) (e.g., FIG. 22B).

Process 1016 includes identifying a corresponding plurality of reference points on the 3D image, e.g., CT scan. This may include identifying the reference points on each segment of a plurality of segments of the 3D image. In correspondence with the 2D images, the reference points may include the left and right acetabular CoRs 1114L and 1114R (FIG. 23A), the left and right anterior superior iliac crests 1110L and 1110R (FIG. 23B), and the pubic symphysis 1112 (FIG. 23C).

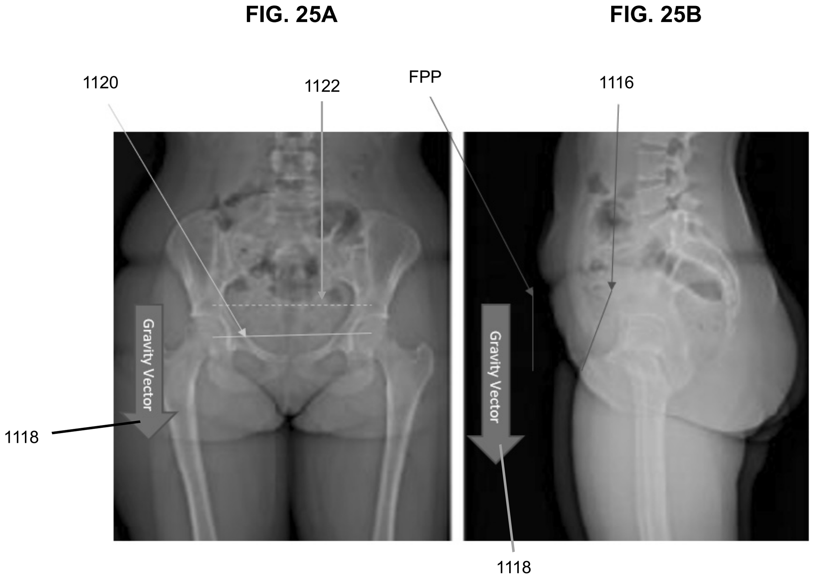

Processes 1018, 1020, and 1022 include constructing a biomechanical reference, e.g., a biomechanical reference line or reference plane, on each image of the plurality of 2D images. Each biomechanical reference may be based on the plurality of reference points on each respective image. For example, process 1018 may include constructing a biomechanical reference such as, e.g., an anterior pelvic plane (APP) 1116 on the sitting lateral 2D image (see, e.g., FIG. 24). The APP 1116 connects the anterior superior iliac crests 1110R, 1110L and the pubic symphysis 1112 (see, FIG. 22B). The APP is measured relative to a vertical line that extends parallel to the assumed gravity vector 1118. Process 1020 may include constructing a biomechanical reference such as, e.g., a bicoxofemoral line 1120 on the AP 2D image (FIG. 25A). The bicoxofemoral line 1120 connects the left and right CoRs 1114L, 1114R (FIG. 22A). The bicoxofemoral line 1120 is further measured relative to a horizontal line 1122 that extends perpendicularly relative to the assumed gravity vector 1118. Process 1022 may include constructing a biomechanical reference such as, e.g., an APP 1116 on the 2D lateral image (FIG. 25B), as described relative to process 1018 and the 2D lateral seated image in FIG. 24.

Process 1024 includes constructing a biomechanical reference on each segment of the plurality of segments of the 3D image, based on the plurality of reference points on each respective segment. The biomechanical reference constructed on the 3D image may correspond to those constructed on the 2D images in one or more of processes 1018, 1020, and/or 1022 (see FIGS. 24, 25A, 25B). For example, process 1024 may include constructing biomechanical reference(s) such as an APP 1116 and/or a bicoxofemoral line 1120 on the 3D image using the reference points identified on the 3D image in process 1016 (FIGS. 23A-23C).

Process 1026 includes aligning the corresponding biomechanical references of the plurality of segments of the 3D image with the biomechanical references of the plurality of 2D images to provide a functionally aligned 3D image.

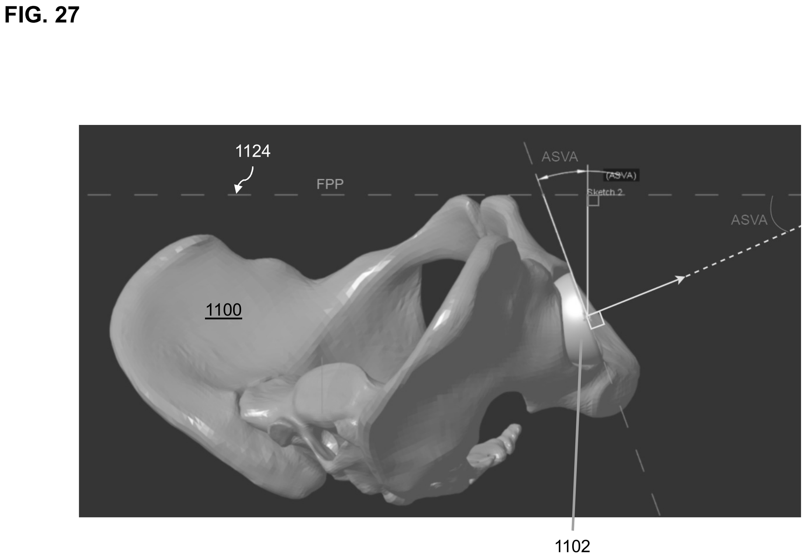

Process 1028 includes identifying a reference line and/or a reference plane on the functionally aligned 3D image. These reference lines and/or planes may then be used to plan aspects of the placement of the acetabular shell 1102. Process 1028 may include identifying the patient's functional pelvic plane (FPP) 1124 (FIG. 27) on the functionally aligned 3D image. The identified FPP 1124 on the functionally aligned 3D image reflects the patient's true FPP, as it is based on the patient's true alignment as captured in the weight-bearing 2D images. In contrast, since the non-weight bearing 3D image represents the patient's alignment as being straight, rather than its native weight-bearing condition, the FPP as determined using the original 3D image would be parallel to the ground. In many patient cases this alignment, and therefore the FPP, may differ from what is returnable from the originally acquired, non-weight bearing 3D image. This may be due to, e.g., inclination in a patient's posture. Alignment of the 3D image to incorporate these aspects of the patient's biomechanics influences the planning of the inclination and version angles for the acetabular shell 1102.

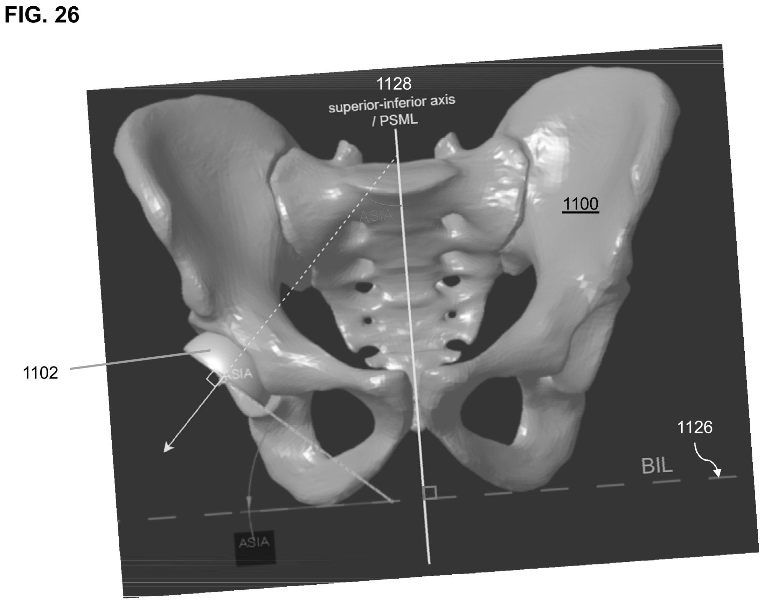

The FPP 1124 may in turn be used to further identify a reference line on the functionally aligned 3D image. The reference line may particularly be determined relative to the FPP 1124. In one example, with reference to FIG. 26, the pelvis 1100 is viewed from the FPP 1124 (labeled in FIG. 27). In this frame of reference, a particular reference line, for example the bi-ischial line (BI line) 1126 (FIG. 15) is identified. The BI line 1126 connects the ischial tuberosities of the pelvis 1100.

Process 1030 includes planning the placement of the acetabular shell 1102 on the functionally aligned 3D image (FIGS. 26, 27). As noted, this may include, e.g., planning an inclination angle of the acetabular shell 1102 relative to the reference line, e.g., relative to the BI line 1126 (FIG. 26), and planning a version angle of the acetabular shell 1102, e.g., relative to the FPP 1124 (FIG. 27). The planning of the acetabular shell 1102 is dependent upon the definition of the FPP 1124 and the coronal alignment. The functional alignment of the 3D image, e.g., in processes 1002 through 1026, enables the determination of the foregoing angles in a manner that is representative of the patient's native biomechanics, resulting in CT planning for the acetabular shell 1102 placement that incorporates consideration of the functional alignment of the patient.

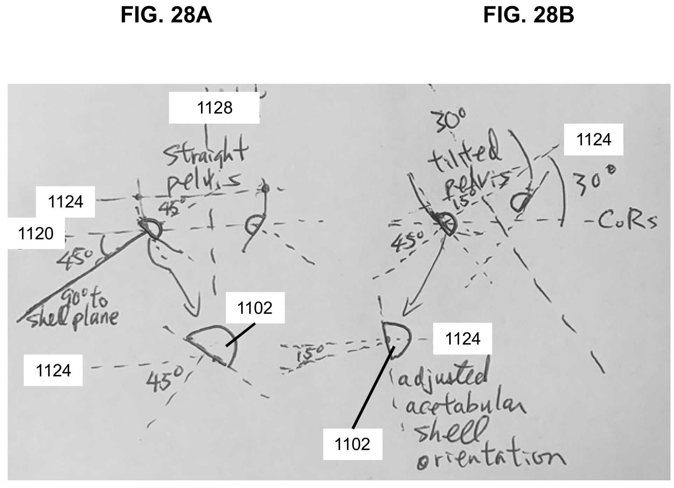

Referring to the example of FIGS. 28A-28B, the contrast in acetabular shell placement plan is illustrated for whose native biomechanics include a tilt to the left (counter clock-wise, or to the patient's right) by about 30°. FIG. 28A illustrates a placement planned using the original 3D image, as compared to the same patient whose acetabular shell placement is planned using the aligned 3D image (FIG. 28B) produced in process 1026 (FIG. 21). FIGS. 28A and 28B equally could be understood to represent the contrast in acetabular shell placement planning for a patient with straight alignment (FIG. 28A) as compared to a patient whose native biomechanics that include a tilt to the left (counter clock-wise, or to the patient's right) by about 30° (FIG. 28B).

In FIG. 28A, the FPP 1124 is parallel to the floor, and is perpendicular to the superior-inferior axis 1128. The angle perpendicular to the acetabular shell plane relative to the FPP (1124), or the bicoxofemoral line 1120, is about 45°. In FIG. 28B, as noted, the patient's pelvis is tilted to the left (counter clock-wise, or to the patient's right) by about 30°. The plane of the acetabular shell 1102 will therefore be rotated right by 30°. The angle perpendicular to the acetabular shell plane relative to the FPP (1124), or the bicoxofemoral line 1120, is about 15°.

### Further Definitions and Embodiments

In the above description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected," "coupled," "responsive," or variants thereof to another element, it can be directly connected, coupled, or responsive to the other element or intervening elements may be present. In contrast, when an element is referred to as being "directly connected," "directly coupled," "directly responsive," or variants thereof to another element, there are no intervening elements present. Like numbers refer to like elements throughout. Furthermore, "coupled," "connected," "responsive," or variants thereof as used herein may include wirelessly coupled, connected, or responsive. As used herein, the singular forms "a," "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways.

It is to be understood that the phraseology and terminology used herein is for the purpose of description and should not be regarded as limiting. The use of "comprise," "comprising," "comprises," "include," "including," "includes," "have," "has," "having," or variations thereof, is meant to encompass the items listed thereafter and equivalents thereof as well as additional items in an open-ended manner. The use of such terms includes one or more stated features, integers, elements, steps, components, or functions, but does not preclude the presence or addition of one or more other features, integers, elements, steps, components, functions, or groups thereof.

Unless specified or limited otherwise, the terms "mounted," "connected," "attached," "supported," and "coupled" and variations thereof are used broadly and encompass both direct and indirect mountings, connections, attachments, supports, and couplings. Further, "connected" and "coupled" are not restricted to physical or mechanical connections or couplings.

As used herein, the term "instrument" is used in a non-limiting manner and can be used interchangeably with "tool" and "implant" to generally refer to any type of device that can be used during a surgical procedure in accordance with embodiments disclosed herein. The more general term, device, can also refer to structure of the end effector, etc. Example instruments, tools, and implants include, without limitation, reamer constructs, drills, screwdrivers, saws, dilators, retractors, probes, implant inserters, and implant devices such as shells and trial shells, screws, spacers, interbody fusion devices, plates, rods, etc. The term "end effector" may be used interchangeably with the terms "end effectuator" and "effectuator element."

Furthermore, as used herein, the common abbreviation "e.g.," which derives from the Latin phrase "exempli gratia," may be used to introduce or specify a general example or examples of a previously mentioned item, and is not intended to be limiting of such item. The common abbreviation "i.e.," which derives from the Latin phrase "id est," may be used to specify a particular item from a more general recitation.

The invention could be defined inter alia by the following examples:
1. A method for planning a placement of an acetabular shell in a total hip arthroplasty (THA) procedure, comprising: identifying a plurality of reference points on each of a plurality of two-dimensional (2D) images depicting a functional alignment of a pelvis of a patient in a weight-bearing position; identifying a corresponding plurality of reference points on a three-dimensional (3D) image depicting the pelvis of the patient in a non-weight bearing position; constructing a biomechanical reference on each of the 2D images based on the identified plurality of reference points on each respective 2D image; constructing a corresponding biomechanical reference on the 3D image based on the identified corresponding reference points on the 3D image; aligning the corresponding biomechanical reference of the 3D image with the biomechanical references of the 2D images to provide a functionally aligned 3D image for use in planning the placement of the acetabular shell in the patient.
2. The method of Example 1, wherein the plurality of 2D images includes: a 2D anterior-posterior (AP) image of the patient in a standing position; a 2D lateral image of the patient in a seated position; and a 2D lateral image of the patient in a standing position.
3. The method of Example 1, wherein the plurality of reference points comprises a left and a right acetabular center of rotation (CoR).
4. The method of Example 3, wherein the biomechanical reference comprises a bicoxofemoral line connecting the left and the right acetabular CoRs.
5. The method of Example 1, wherein the plurality of reference points comprises a left and a right anterior superior iliac crest and a pubic symphysis.
6. The method of Example 5, wherein the biomechanical reference comprises an anterior pelvic plane (APP) connecting the left and the right anterior superior iliac crests and the pubic symphysis.
7. The method of Example 1, further comprising, after the aligning, identifying a functional pelvic plane (FPP) of the patient on the functionally aligned 3D image.
8. The method of Example 7, further comprising identifying a reference line on the functionally aligned 3D image, wherein the reference line is determined relative to the FPP.
9. The method of Example 8, wherein the planning comprises planning an inclination angle of the acetabular shell relative to the reference line, wherein the reference line is a bi-ischial (BI) line.
10. The method of Example 7, wherein the planning comprises planning a version angle of the acetabular shell relative to the FPP.
11. The method of Example 1, wherein each image in the plurality of 2D images comprises an x-ray image.
12. The method of Example 1, wherein the 3D image comprises a computed tomography (CT) scan.
13. The method of Example 12, further comprising, prior to identifying the corresponding plurality of reference points, segmenting the 3D image to yield a segmented three-dimensional (3D) image, wherein each segment of the plurality of segments corresponds to a 2D slice of the CT scan.
14. A method of aligning a three-dimensional (3D) image of a pelvis of a patient to a native functional alignment of the patient, comprising: acquiring the 3D image of the pelvis in a non-weight bearing position, and segmenting the 3D image; acquiring a plurality of two-dimensional (2D) images depicting a native functional alignment of the pelvis in a plurality of weight-bearing positions; identifying a plurality of reference points on each image of the 2D images; identifying a corresponding plurality of reference points on the segmented 3D image;
   constructing a biomechanical reference on each of the 2D images based on the identified plurality of reference points on each respective image; constructing a corresponding biomechanical reference on the segmented 3D image based on the identified corresponding reference points on the segmented 3D image; and aligning the corresponding biomechanical reference of the segmented 3D image with the biomechanical references of the 2D images to provide a functionally aligned 3D image for use in planning the placement of the acetabular shell in the patient.
15. The method of Example 14, wherein the plurality of 2D images includes: a 2D anterior-posterior (AP) image of the patient in a standing position; a 2D lateral image of the patient in a seated position; and a 2D lateral image of the patient in a standing position.
16. The method of Example 14, wherein the set of reference points comprises a left and a right acetabular center of rotation (CoR), and the biomechanical reference comprises a bicoxofemoral line connecting the left and the right acetabular CoRs.
17. The method of Example 14, wherein the set of reference points comprises a left and a right anterior superior iliac crest and a pubic symphysis, and the biomechanical reference comprises an anterior pelvic plane (APP) connecting the left and the right anterior superior iliac crests and the pubic symphysis.
18. The method of Example 14, wherein each image in the plurality of 2D images comprises an x-ray image.
19. The method of Example 14, wherein the 3D image comprises a computed tomography (CT) scan, and each segment of the plurality of segments corresponds to a 2D slice of the CT scan.
20. A system for computer assisted navigation during a total hip arthroplasty (THA) surgery, comprising a computer platform including a processor and a memory, operative to: identify a plurality of reference points on each of a plurality of two-dimensional (2D) images depicting a functional alignment of a pelvis of a patient in a weight-bearing position; identify a corresponding plurality of reference points on a segmented three-dimensional (3D) image depicting the pelvis in a non-weight bearing position; construct a biomechanical reference on each of the 2D images based on the identified plurality of reference points on each respective 2D image; construct a corresponding biomechanical reference on the segmented 3D image based on the identified corresponding reference points on the segmented 3D image; align the corresponding biomechanical references of the segmented 3D image with the biomechanical references of the 2D images to provide a functionally aligned 3D image for use in planning the placement of the acetabular shell on the functionally aligned segmented 3D image.

## Claims

1. A method for planning a placement of an acetabular shell in a total hip arthroplasty (THA) procedure, comprising:
- identifying a plurality of reference points on each of a plurality of two-dimensional (2D) images depicting a functional alignment of a pelvis of a patient in a weight-bearing position;
- identifying a corresponding plurality of reference points on a three-dimensional (3D) image depicting the pelvis of the patient in a non-weight bearing position;
- constructing a biomechanical reference on each of the 2D images based on the identified plurality of reference points on each respective 2D image;
- constructing a corresponding biomechanical reference on the 3D image based on the identified corresponding reference points on the 3D image;
- aligning the corresponding biomechanical reference of the 3D image with the biomechanical references of the 2D images to provide a functionally aligned 3D image for use in planning the placement of the acetabular shell in the patient.

2. The method of claim 1, wherein the plurality of 2D images includes:
- a 2D anterior-posterior (AP) image of the patient in a standing position;
- a 2D lateral image of the patient in a seated position; and
- a 2D lateral image of the patient in a standing position.

3. The method of claim 1 or 2, wherein the plurality of reference points comprises a left and a right acetabular center of rotation (CoR).

4. The method of any one of the preceding claims, wherein the biomechanical reference comprises a bicoxofemoral line connecting the left and the right acetabular CoRs.

5. The method of any one of the preceding claims, wherein the plurality of reference points comprises a left and a right anterior superior iliac crest and a pubic symphysis.

6. The method of any one of the preceding claims, wherein the biomechanical reference comprises an anterior pelvic plane (APP) connecting the left and the right anterior superior iliac crests and the pubic symphysis.

7. The method of any one of the preceding claims, further comprising, after the aligning, identifying a functional pelvic plane (FPP) of the patient on the functionally aligned 3D image.

8. The method of claim 7, further comprising identifying a reference line on the functionally aligned 3D image, wherein the reference line is determined relative to the functional pelvic plane (FPP).

9. The method of any one of the preceding claims, wherein the planning comprises planning an inclination angle of the acetabular shell relative to the reference line, wherein the reference line is a bi-ischial (BI) line.

10. The method of claim 7, wherein the planning comprises planning a version angle of the acetabular shell relative to the functional pelvic plane (FPP).

11. The method of any one of the preceding claims, wherein each image in the plurality of 2D images comprises an x-ray image.

12. The method of any one of the preceding claims, wherein the 3D image comprises a computed tomography (CT) scan.

13. The method of claim 12, further comprising, prior to identifying the corresponding plurality of reference points, segmenting the 3D image to yield a segmented three-dimensional (3D) image,
wherein each segment of the plurality of segments corresponds to a 2D slice of the CT scan.

14. A method of aligning a three-dimensional (3D) image of a pelvis of a patient to a native functional alignment of the patient, comprising:
- acquiring the 3D image of the pelvis in a non-weight bearing position, and segmenting the 3D image;
- acquiring a plurality of two-dimensional (2D) images depicting a native functional alignment of the pelvis in a plurality of weight-bearing positions;
- identifying a plurality of reference points on each image of the 2D images;
- identifying a corresponding plurality of reference points on the segmented 3D image;
- constructing a biomechanical reference on each of the 2D images based on the identified plurality of reference points on each respective image;
- constructing a corresponding biomechanical reference on the segmented 3D image based on the identified corresponding reference points on the segmented 3D image; and
- aligning the corresponding biomechanical reference of the segmented 3D image with the biomechanical references of the 2D images to provide a functionally aligned 3D image for use in planning the placement of the acetabular shell in the patient.

15. A system for computer assisted navigation during a total hip arthroplasty (THA) surgery, comprising a computer platform including a processor and a memory, operative to:
- identify a plurality of reference points on each of a plurality of two-dimensional (2D) images depicting a functional alignment of a pelvis of a patient in a weight-bearing position;
- identify a corresponding plurality of reference points on a segmented three-dimensional (3D) image depicting the pelvis in a non-weight bearing position;
- construct a biomechanical reference on each of the 2D images based on the identified plurality of reference points on each respective 2D image;
- construct a corresponding biomechanical reference on the segmented 3D image based on the identified corresponding reference points on the segmented 3D image;
- align the corresponding biomechanical references of the segmented 3D image with the biomechanical references of the 2D images to provide a functionally aligned 3D image for use in planning the placement of the acetabular shell on the functionally aligned segmented 3D image.
